# EUROPEAN PATENT APPLICATION

(11) **EP 3 150 219 A1**
(43) Date of publication of application: **05.04.2017**
(21) Application number: 16174665.6
(22) Date of filing: 15.05.2009
(51) Int. Cl.: A61K 38/22, C07K 14/64, A61P 9/04

(54) **METHOD OF TREATING CHRONIC HEART FAILURE**

(30) Priority: 16.05.2008 US 127889 P; 28.08.2008 US 190545 P; 08.12.2008 US 201240 P
(62) Divisional of application: 09747738.4
(71) Applicant: Corthera, Inc., San Carlos, CA 94070 (US)
(72) Inventor: UNEMORI, Elaine, Oakland, CA 94610 (US); TEICHMAN, Sam, L., Alamo, CA 94507 (US); DSCHIETZIG, Thomas, San Mateo, CA 94402 (US); STEWART, Dennis, R., Los Gatos, CA 95033 (US); WHITEHOUSE, Martha Jo, San Mateo, CA 94402 (US)
(74) Representative: Westberg, Marie Suzanne

(57) **Abstract**

The present disclosure relates to methods for treating human subjects afflicted with chronic heart failure. The methods described herein employ administration of relaxin.

## Description

This application claims benefit under 35 U.S.C. 119(e) of U.S. Provisional Patent Application Nos. 61/201,240, filed December 8, 2008; 61/190,545, filed August 28, 2008; and 61/127,889, filed May 16, 2008, all of which are incorporated herein by reference in their entirety for all purposes.

### FIELD

The present disclosure relates to methods for treating human subjects afflicted with chronic heart failure. The methods described herein employ administration of relaxin.

### BACKGROUND

Heart failure is a major health problem and is the most frequent cause of hospitalization in patients older than 65 years (Krumholz et al., Am. Heart J., 139: 72-7, 2000). The fundamental symptoms of heart failure are dyspnea, fatigue and fluid retention, which can lead to pulmonary congestion and peripheral edema. Heart failure is almost always a progressive disease, and is easily exacerbated resulting in acute decompensated heart failure (Hunt et al., Circulation, 112: 154-235, 2005). Acute heart failure (AHF) is the single most costly hospital admission diagnosis according to a recent presentation from the Center for Medicare and Medicaid Administration. In fact, AHF accounts for more than one million hospitalizations per year, with a hospitalization readmission rate within six months of nearly fifty percent (Koelling et al., Am Heart J, 147: 74-8, 2004).

While significant advances have been made in the realm of chronic heart failure (HF) management, it is still associated with considerable morbidity and mortality. The median life expectancy for symptomatic patients is less than five years, and one-year mortality rates of up to 90% are reported for patients with most advanced disease (Stewart et al., Eur J Heart Fail, 3: 315-322, 2001; Hershberger et al., J Card Fail, 9: 180-187, 2003; and Rose et al., N Eng J Med, 345: 1435-1443, 2001). Accordingly, the goal of clinical management of heart failure is to extend the compensated (stabilized) period and to prevent progression of the disease for as long as possible. There is now an increasing awareness of the complex interplay that occurs between the heart and kidneys among patients with heart failure. As such, many of the traditional therapeutics used to treat this patient population and which can significantly alter renal function are no longer considered to be optimal treatment options. Moreover, current medications used to manage chronic heart failure patients have limited effectiveness or serious side effects such as hypotension, tachycardia, arrhythmia and worsening renal failure. Thus, the development of new drugs and treatment regimens that are capable of stabilizing patients with chronic HF and are accompanied less adverse side effects is desirable.

### BRIEF SUMMARY OF THE PREFERRED EMBODIMENTS

The present disclosure relates to methods for treating human subjects afflicted with chronic heart failure. The methods described herein employ administration of relaxin. The present disclosure provides methods for treating patients with congestive heart failure (CHF) by administering relaxin. The number of hospital admissions due to deterioration of CHF is on the rise and the cost associated with caring for these patients is staggering. Thus, a new therapeutic approach is needed and the disclosure addresses this need. One advantage of the disclosure is that the administration of relaxin results in a balanced vasodilation that prevents compensated heart failure from developing into acute decompensated heart failure. As such, the subjects can be maintained at a steady-state level where hospitalization is not required and the number or duration of hospital visits is significantly reduced. Another advantage of the present disclosure is that relaxin, when administered to patients, shows effectiveness with little to no adverse drug reactions (ADRs). Herein, relaxin is shown to have a beneficial effect on reducing acute decompensation by stabilizing patients without causing ADRs. Thus, the present disclosure provides a treatment that leads to balanced vasodilation in a specific patient population that suffers from chronic HF.

One aspect of the disclosure provides a method of reducing acute cardiac decompensation events including selecting a human subject with chronic HF, wherein the subject has a vasculature and the vasculature has relaxin receptors. The method further includes administering to the subject a pharmaceutical formulation including pharmaceutically active relaxin in an amount effective to reduce frequency of acute cardiac decompensation events in the subject by binding to the relaxin receptors in the vasculature of the subject, resulting in balanced vasodilation. The cardiac decompensation can be due to any one or more causes, including but not limited to, neurohormonal imbalance, fluid overload, cardiac arrhythmia, and cardiac ischemia. In one embodiment, the human subject suffers from acute vascular failure.

Relaxin employed in the pharmaceutical formulations of the disclosure can be, for example, synthetic or recombinant relaxin, or a pharmaceutically effective relaxin agonist. In one embodiment of the disclosure, relaxin is H1 human relaxin. In another embodiment, relaxin is H2 human relaxin. In yet another embodiment, relaxin is H3 human relaxin. In a further embodiment, relaxin is synthetic or recombinant human relaxin, or a pharmaceutically effective relaxin agonist. Thus, the subject can be treated with a pharmaceutical formulation of synthetic or recombinant human relaxin or relaxin agonist. In one embodiment of the disclosure, the subject is treated with synthetic human relaxin. In another embodiment, the subject is treated with recombinant human relaxin. In yet another embodiment, the subject is treated with a pharmaceutically effective relaxin agonist. Relaxin can be administered to the subject through a number of different routes, including but not limited to, intravenously, subcutaneously, intramuscularly, sublingually and via inhalation. More specifically, the pharmaceutical formulation of relaxin or relaxin agonist can be administered to the subject in an amount in a range of about 10 to 1000 µg/kg of subject body weight per day. As such, relaxin is administered to the subject so as to maintain a serum concentration of relaxin of from about 1 to 500 ng/ml.

Human subjects that would benefit from the methods of the disclosure were diagnosed with heart failure about a year or more prior to administration of relaxin. Acute cardiac decompensation events, whose frequency can be reduced by relaxin treatment include but are not limited to, dyspnea, hypertension, arrhythmia, reduced renal blood flow, and renal insufficiency. These events are often associated with admission or re-admission to a hospital. In one embodiment of the disclosure, these acute cardiac decompensation events are pathophysiological in nature. Most commonly, such events are associated with acute decompensated heart failure (AHF). In one embodiment, the human subject suffers from vascular failure. In another embodiment, the acute cardiac decompensation is intermittent.

Another aspect of the disclosure provides a method of reducing frequency of acute cardiac decompensation events. In some embodiments, the methods comprise selecting a human subject with compensated CHF, wherein the subject has a vasculature and the vasculature has relaxin receptors; and administering to the subject a pharmaceutical formulation including pharmaceutically active relaxin in an amount effective to reduce the frequency of acute cardiac decompensation events experienced by the subject by binding to the relaxin receptors in the vasculature of the subject. In this method, treatment with relaxin results in a reduction in frequency of acute cardiac decompensation events, and this effect lasts for at least about 1 to 14 days from onset of relaxin treatment. The acute cardiac decompensation events include, but are not limited to dyspnea, extra body weight due to retention of fluids, length of hospital stay, likelihood of hospital re-admission, need for loop diuretics, need for intravenous (IV) nitroglycerin, and an incidence of worsening heart failure. In one embodiment, the patients are treated with relaxin for 48 hours. In another embodiment, the patients are treated with relaxin for 24 hours. In yet another embodiment, the patients are treated with relaxin for 12 hours. In still another embodiment, the patients are treated with relaxin for 6 hours. The effects of relaxin can be measured at any time point, for example, at 1 day, 2 days, 3 days, 4 days, 5 days, 6 days, 7 days, 8 days , 9 days, 10 days, 11 days, 12 days, 13 days, 14 days or later post relaxin administration.

In one preferred embodiment, relaxin is administered at about 30 mcg/kg/day. In one preferred embodiment, relaxin is administered at about 30 mcg/kg/day. In another preferred embodiment, relaxin is administered at about 35 mcg/kg/day. In another preferred embodiment, relaxin is administered at about 40 mcg/kg/day. In another preferred embodiment, relaxin is administered at about 45 mcg/kg/day. In another preferred embodiment, relaxin is administered at about 50 mcg/kg/day. In another preferred embodiment, relaxin is administered at about 55 mcg/kg/day. In another preferred embodiment, relaxin is administered at about 60 mcg/kg/day. In another preferred embodiment, relaxin is administered at about 65 mcg/kg/day. In another preferred embodiment, relaxin is administered at about 70 mcg/kg/day. In another preferred embodiment, relaxin is administered at about 75 mcg/kg/day. In another preferred embodiment, relaxin is administered at about 80 mcg/kg/day. In another preferred embodiment, relaxin is administered at about 85 mcg/kg/day. In another preferred embodiment, relaxin is administered at about 100 mcg/kg/day. Relaxin may also be administered at a dosage of 90 to 200 mcg/kg/day. Pharmaceutically effective relaxin includes recombinant or synthetic H1 human relaxin, H2 human relaxin or H3 human relaxin or an agonist or a variant thereof. In one preferred embodiment, relaxin is administered to the subject so as to maintain a serum concentration of about 10 ng/ml. The pharmaceutical formulation of relaxin can be administered intravenously, subcutaneously, intramuscularly, sublingually or via inhalation. In one preferred embodiment, the pharmaceutical formulation of relaxin is administered intravenously. The relaxin receptors are activated through the binding of relaxin and include, but are not limited to, LRG7, LGR8, GPCR135, and GPCR142. The binding of relaxin to the relaxin receptors triggers the production of nitric oxide (NO) which results in balanced vasodilation. The relaxin receptors are located, for example, on the smooth muscle tissue of the vasculature.

In addition, the present disclosure provides a method of treating heart failure, comprising administering to a human subject a pharmaceutically active relaxin in an amount therapeutically effective to reduce frequency or duration of hospitalization of the subject compared to treatment without relaxin, wherein the subject has Class II, or Class III heart failure according to New York Heart Association (NYHA) classification of heart failure at onset of the administering. In one embodiment, the method comprises reducing the frequency of decompensation compared to treatment without relaxin. In another embodiment, the decompensation comprises a symptom requiring unscheduled medical care selected from the group consisting of dyspnea, edema, and fatigue. In another embodiment, the decompensation comprises one or more of increased fluid retention, hypotension, hypertension, arrhythmia, reduced renal blood flow, elevated levels of brain natriuretic peptide (BNP), elevated levels of N-terminal pro-B-type natriuretic peptide (NT-proBNP), elevated levels of blood urea nitrogen (BUN) and elevated levels of creatinine. In another embodiment, the decompensation requires administration of an intravenous diuretic. In another embodiment, the decompensation comprises reducing risk of death due to heart failure, wherein the subject has Stage B, or Stage C, structural heart disease, according to American Heart Association guidelines, without current symptoms of heart failure at the onset of the administering. In another embodiment, the subject has Stage C, structural heart disease, according to American Heart Association guidelines, with current symptoms of heart failure at the onset of the administering. In yet another embodiment, the subject has a left ventricular ejection fraction (LVEF) of about 35% or less at the onset of the administering. In yet another embodiment, the subject has a systolic blood pressure of about 85 mm Hg or greater at the onset of the administering. In another embodiment, the subject has a systolic blood pressure of between about 85 and 125 mm Hg at the onset of the administering. In one embodiment, the relaxin (e.g., recombinant, purified or synthetic) is H2 human relaxin (or in alternative embodiments, H1 human relaxin or H3 human relaxin). In another embodiment, the relaxin is a relaxin agonist. In one embodiment, the relaxin is administered at a fixed dose of between about 10 and 960 (e.g., about 10, 30, 100, 240, 480 or 940) mcg/kg/day (without prior titration). In another embodiment, the relaxin is administered using a route of delivery selected from the group consisting of intravenous, intramuscular, and subcutaneous (or by intradermal, sublingual, inhalation, or wearable infusion pump). In yet another embodiment, the relaxin is administered by infusion for a time period selected from the group consisting of at least about 4, 8, 12, 24 and 48 hours. In another embodiment, the administering comprises continuous administration of the relaxin. In yet another embodiment, the relaxin is administered by injection at a frequency selected from the group consisting of thrice daily, twice daily, once daily, thrice weekly, twice weekly, once weekly, bi-weekly, and monthly. In one embodiment, the administering comprises intermittent administration of the relaxin. In another embodiment, the administering does not result in an adverse effect selected from the group consisting of hypotension, tachycardia, arrhythmia, and worsening renal function. In another embodiment, the administering further results in decreasing one or more of systemic vascular resistance, pulmonary capillary wedge pressure, pulmonary vascular resistance, blood urea nitrogen, creatinine, and circulating N-terminal prohormone brain natriuretic peptide. In another embodiment, the subject is receiving one or more of an anti-platelet, a beta-blocker, a diuretic, and an anti-angiotensin therapy (angiotensin-converting enzyme inhibitor or angiotensin receptor blocker) at the onset of the administering. In yet another embodiment, the subject does not have acute heart failure requiring hospitalization at the onset of the administering.

The disclosure also provides a method of treating heart failure, comprising administering to a human subject a pharmaceutically active relaxin in an amount therapeutically effective to improve functional capacity of the subject, wherein the subject has Class III, or Class IV heart failure according to New York Heart Association (NYHA) classification of heart failure. In one embodiment, the improved functional capacity corresponds to a higher score on a Minnesota Living With Heart Failure@ Questionnaire (or similar assessment of quality of life or impact of physical heart failure symptoms on social, mental and/or emotional functions). In another embodiment, the improved functional capacity corresponds to an increased distance traveled in a 6-minute walk test (or similar measurement of exercise tolerance). In one embodiment, the improved functional capacity corresponds to an increase in maximal oxygen consumption (VO₂max). In another embodiment, the improved functional capacity corresponds to a change to a more mild class of heart failure according to the NYHA classification of heart failure. In another embodiment, the subject has Stage C, structural heart disease, according to American Heart Association guidelines, with current symptoms of heart failure at the onset of the administering. In yet another embodiment, the subject has Stage D, refractory heart failure, according to American Heart Association guidelines, characterized by marked heart failure symptoms at rest despite optimal medical therapy at the onset of the administering. In yet another embodiment, the subject has Stage D heart failure and is eligible for one or both of mechanical circulatory support and cardiac transplantation. In another embodiment, the subject has Stage D heart failure and is eligible for end-of-life care. In yet another embodiment, the subject was diagnosed with heart failure at least one year prior to the onset of the administering. In one embodiment, the relaxin (e.g., recombinant, purified or synthetic) is H2 human relaxin (or in alternative embodiments, H1 human relaxin or H3 human relaxin). In another embodiment, the relaxin is a relaxin agonist. In one embodiment, the relaxin is administered at a fixed dose of between about 10 and 960 (e.g., about 10, 30, 100, 240, 480 or 940) mcg/kg/day (without prior titration). In another embodiment, the relaxin is administered at a fixed dose of between about 240 and 960 (e.g., about 240, 480 or 940) mcg/kg/day (without prior titration). In one embodiment, the relaxin is administered using a route of delivery selected from the group consisting of intravenous, intramuscular, and subcutaneous (or by intradermal, sublingual, inhalation, or wearable infusion pump). In another embodiment, the relaxin is administered by infusion for a time period selected from the group consisting of at least about 4, 8, 12, 24 and 48 hours. In yet another embodiment, the administering comprises continuous administration of the relaxin. In yet another embodiment, the relaxin is administered by injection at a frequency selected from the group consisting of thrice daily, twice daily, once daily, thrice weekly, twice weekly, once weekly, bi-weekly, and monthly. In one embodiment, the administering comprises intermittent administration of the relaxin. In another embodiment, the administering does not result in an adverse effect selected from the group consisting of hypotension, tachycardia, arrhythmia, and worsening renal function. In another embodiment, the administering further results in decreasing one or more of systemic vascular resistance, pulmonary capillary wedge pressure, pulmonary vascular resistance, blood urea nitrogen, creatinine, and circulating N-terminal prohormone brain natriuretic peptide. In another embodiment, the subject is receiving one or more of an anti-platelet, a beta-blocker, a diuretic, and an anti-angiotensin therapy (angiotensin-converting enzyme inhibitor or angiotensin receptor blocker) at the onset of the administering. In yet another embodiment, the subject does not have acute heart failure requiring hospitalization at the onset of the administering.

Another aspect of the disclosure embodies a method of treating heart failure, comprising administering to a human subject a pharmaceutically active relaxin in an amount therapeutically effective to reduce use of concurrent chronic heart failure medications taken by the subject, wherein the concurrent chronic heart failure medications comprise one or more of an anti-platelet, a beta-blocker, a diuretic, and an anti-angiotensin therapy (angiotensin-converting enzyme inhibitor or angiotensin receptor blocker). In one embodiment, the subject has Class II or Class III heart failure according to New York Heart Association (NYHA) classification of heart failure at the onset of the administering. In another embodiment, the subject has Stage B or Stage C, structural heart disease, according to American Heart Association guidelines, without current symptoms of heart failure at the onset of the administering. In yet another embodiment, the subject has Stage C, structural heart disease, according to American Heart Association guidelines, with current symptoms of heart failure at the onset of the administering. In one embodiment, the subject has a left ventricular ejection fraction (LVEF) of about 35% or less at the onset of the administering. In another embodiment, the subject has a systolic blood pressure of about 85 mm Hg or greater at the onset of the administering. In yet another embodiment, the subject has a systolic blood pressure of between about 85 and 125 mm Hg at the onset of the administering. In another embodiment, the relaxin (e.g., recombinant, purified or synthetic) is H2 human relaxin (or in alternative embodiments, H1 human relaxin or H3 human relaxin). In yet another embodiment, the relaxin is a relaxin agonist. In one embodiment, the relaxin is administered at a fixed dose of between about 10 and 960 (e.g., about 10, 30, 100, 240, 480 or 940) mcg/kg/day (without prior titration). In another embodiment, the relaxin is administered using a route of delivery selected from the group consisting of intravenous, intramuscular, and subcutaneous (or by intradermal, sublingual, inhalation, or wearable infusion pump). In yet another embodiment, the relaxin is administered by infusion for a time period selected from the group consisting of at least about 4, 8, 12, 24 and 48 hours. In another embodiment, the administering comprises continuous administration of the relaxin. In yet another embodiment, the relaxin is administered by injection at a frequency selected from the group consisting of thrice daily, twice daily, once daily, thrice weekly, twice weekly, once weekly, bi-weekly, and monthly. In one embodiment, the administering comprises intermittent administration of the relaxin. In another embodiment, the administering does not result in an adverse effect selected from the group consisting of hypotension, tachycardia, arrhythmia, and worsening renal function. In another embodiment, the administering further results in decreasing one or more of systemic vascular resistance, pulmonary capillary wedge pressure, pulmonary vascular resistance, blood urea nitrogen, creatinine, and circulating N-terminal prohormone brain natriuretic peptide. In another embodiment, the reduction in use comprises a reduction in dose of one or more of the concurrent chronic heart failure medications. In another embodiment, the reduction in use comprises a discontinuation of one or more the concurrent chronic heart failure medications. In yet another embodiment, the subject does not have acute heart failure requiring hospitalization at the onset of the administering.

The disclosure further provides a method of treating heart failure, comprising administering to a human subject a pharmaceutically active relaxin in an amount therapeutically effective to increase cardiac index of the subject, wherein the subject has heart failure and the cardiac index of the subject at onset of the administering is less than about 2.5 L/min/m². In another embodiment, the subject has Class II or Class III heart failure according to New York Heart Association (NYHA) classification of heart failure at the onset of the administering. In another embodiment, the cardiac index of the subject is between about 1.8 and 2.5 L/min/m² at the onset of the administering. In yet another embodiment, the subject has a left ventricular ejection fraction (LVEF) of about 35% or less at the onset of the administering. In another embodiment, the subject has a systolic blood pressure of about 85 mm Hg or greater at the onset of the administering. In yet another embodiment, the subject has a systolic blood pressure of between about 85 and 125 mm Hg at the onset of the administering. In one embodiment, the relaxin (e.g., recombinant, purified or synthetic) is H2 human relaxin (or in alternative embodiments, H1 human relaxin or H3 human relaxin). In another embodiment, the relaxin is a relaxin agonist. In one embodiment, the relaxin is administered at a fixed dose of between about 10 and 960 (e.g., about 10, 30, 100, 240, 480 or 940) mcg/kg/day (without prior titration). In another embodiment, the relaxin is administered at a fixed dose of between about 240 and 960 (e.g., about 240, 480 or 940) mcg/kg/day (without prior titration). In one embodiment, the relaxin is administered using a route of delivery selected from the group consisting of intravenous, intramuscular, and subcutaneous (or by intradermal, sublingual, inhalation, or wearable infusion pump). In another embodiment, the relaxin is administered by infusion for a time period selected from the group consisting of at least about 4, 8, 12, 24 and 48 hours. In yet another embodiment, the administering comprises continuous administration of the relaxin. In yet another embodiment, the relaxin is administered by injection at a frequency selected from the group consisting of thrice daily, twice daily, once daily, thrice weekly, twice weekly, once weekly, bi-weekly, and monthly. In one embodiment, the administering comprises intermittent administration of the relaxin. In another embodiment, the administering does not result in an adverse effect selected from the group consisting of hypotension, tachycardia, arrhythmia, and worsening renal function. In another embodiment, the administering further results in decreasing one or more of systemic vascular resistance, pulmonary capillary wedge pressure, pulmonary vascular resistance, blood urea nitrogen, creatinine, and circulating N-terminal prohormone brain natriuretic peptide. In another embodiment, the subject is receiving one or more of an anti-platelet, a beta-blocker, a diuretic, and an anti-angiotensin therapy (angiotensin-converting enzyme inhibitor or angiotensin receptor blocker) at the onset of the administering. In yet another embodiment, the subject does not have acute heart failure requiring hospitalization at the onset of the administering.

### BRIEF DESCRIPTION OF THE DRAWINGS

The present disclosure is best understood when read in conjunction with the accompanying figures, which serve to illustrate the preferred embodiments. It is understood, however, that the disclosure is not limited to the specific embodiments disclosed in the figures.

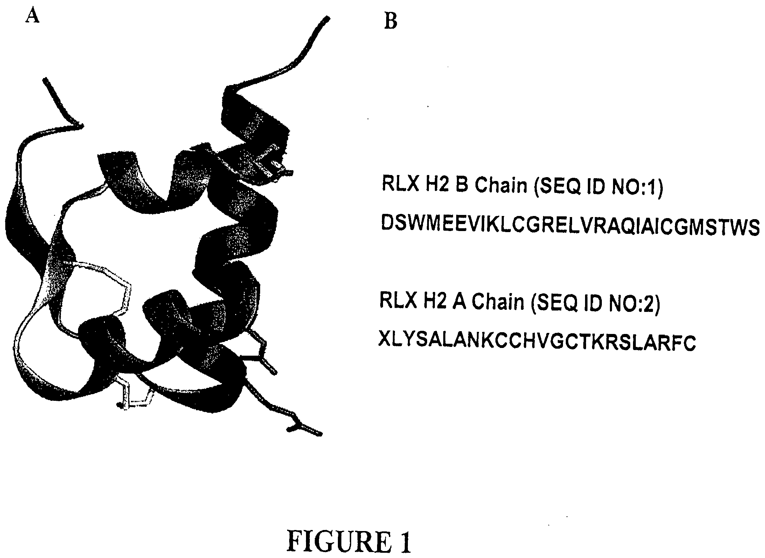

Figure 1A depicts the peptide hormone H2 relaxin which is similar in size and shape to insulin. Figure 1B provides the amino acid sequence of the B chain (SEQ ID NO:1) and the A chain (SEQ ID NO:2 with X representing glutamic acid [E] or glutamine [Q]) of human relaxin 2 (H2).

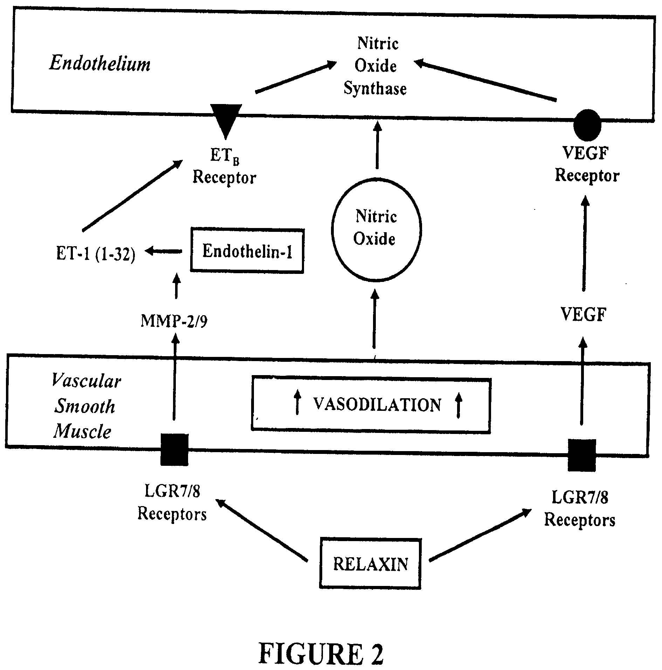

Figure 2 is an illustration of a possible mechanism of action for relaxin. Relaxin receptors LGR7 and LGR8 bind relaxin which activates matrix metalloproteinases MMP-2 and MMP-9 to convert endothelin-1 to truncated endothelin-1 (1-32) which in turn binds to the endothelin B receptor (ET_{B} receptor). This triggers nitric oxide synthase (NOS) to produce nitric oxide (NO) which increases vasodilation.

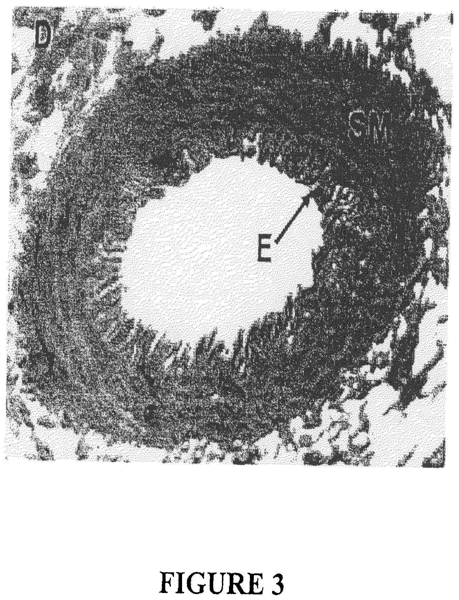

Figure 3 is an illustration of the lumen of a blood vessel. Arrows show the smooth muscle cells (SM) and the endothelium (E). Relaxin receptors are located on the smooth muscle cells of the blood vessels (systemic and renal vasculature).

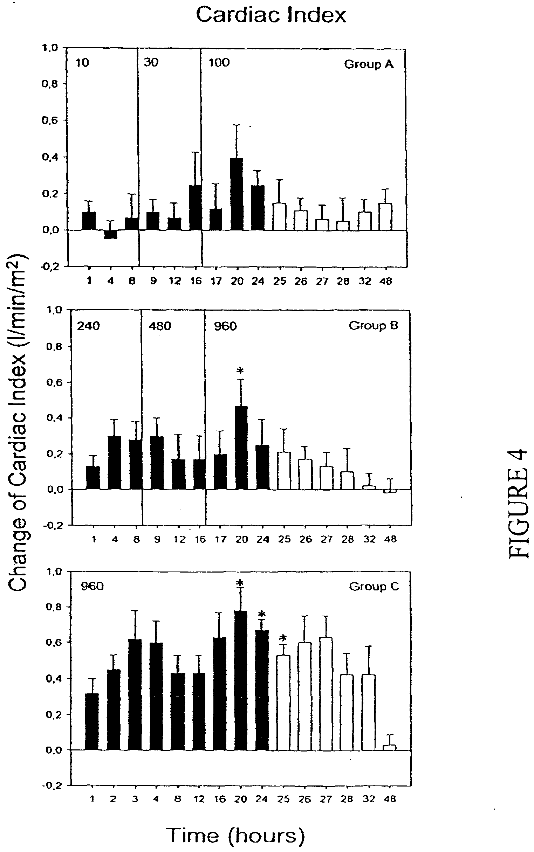

Figure 4 shows cardiac index and relaxin. The graph depicts infusion (black bars) and post-infusion (white bars) for 24 hours (either). Vertical lines mark dosage increases in Groups A and B every 8 hours. Group C received a constant dosage (all in mcg/kg/day). *, P < 0.05 vs. baseline.

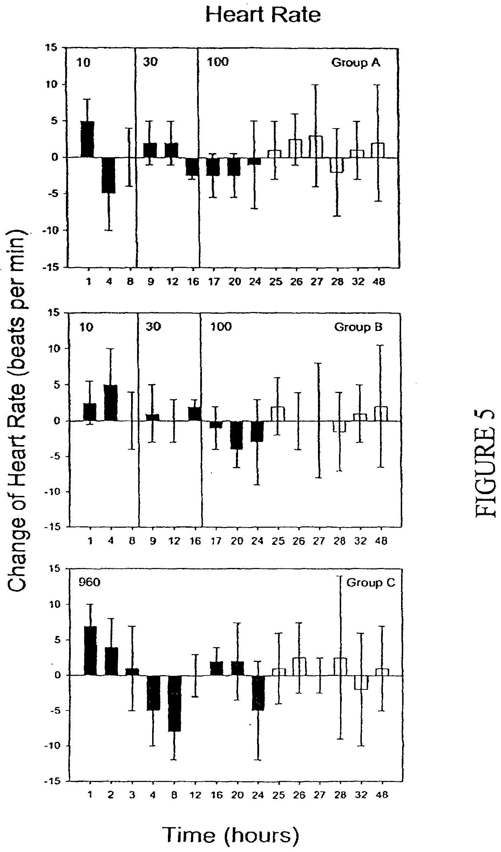

Figure 5 shows heart rate and relaxin. The graph depicts infusion (black bars) and post-infusion (white bars) for 24 hours (either). Vertical lines mark dosage increases in Groups A and B every 8 hours. Group C received a constant dosage (all in mcg/kg/day).

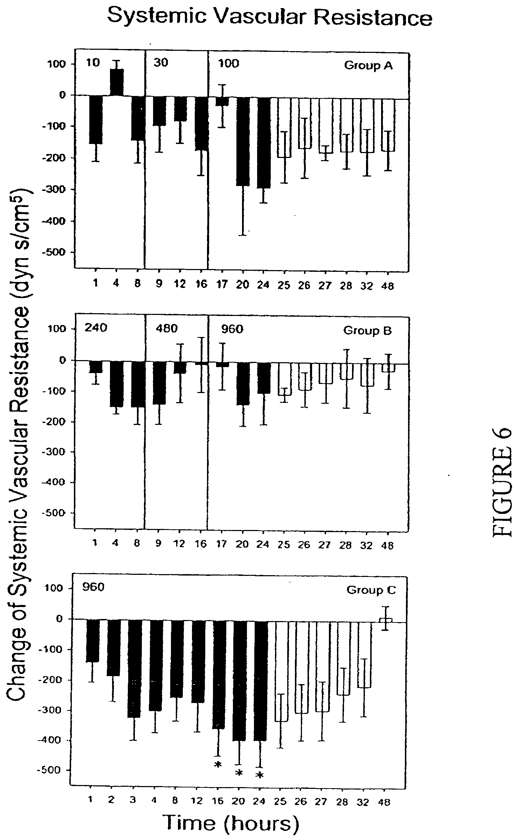

Figure 6 depicts systemic vascular resistance and relaxin. The graph depicts infusion (black bars) and post-infusion (white bars) for 24 hours (either). Vertical lines mark dosage increases in Groups A and B every 8 hours. Group C received a constant dosage (all in mcg/kg/day). *, P < 0.05 vs. baseline.

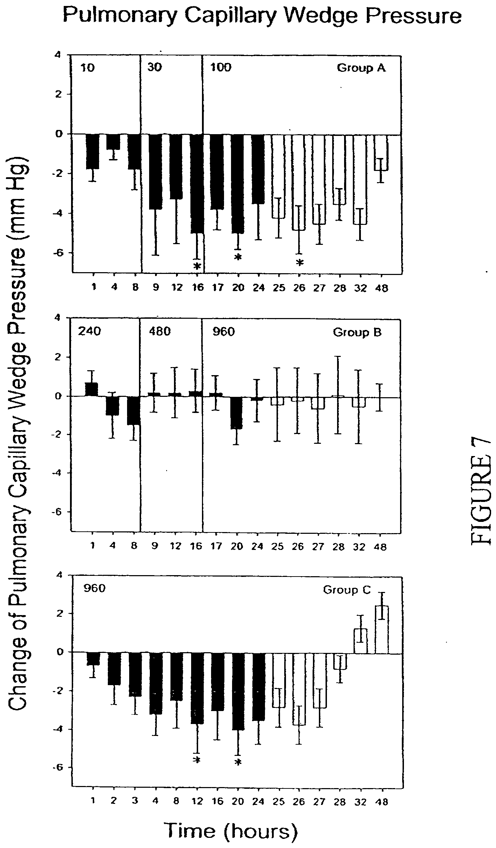

Figure 7 shows pulmonary capillary wedge pressure and relaxin. The graph depicts infusion (black bars) and post-infusion (white bars) for 24 hours (either). Vertical lines mark dosage increases in Groups A and B every 8 hours. Group C received a constant dosage (all in mcg/kg/day). *, P < 0.05 vs. baseline.

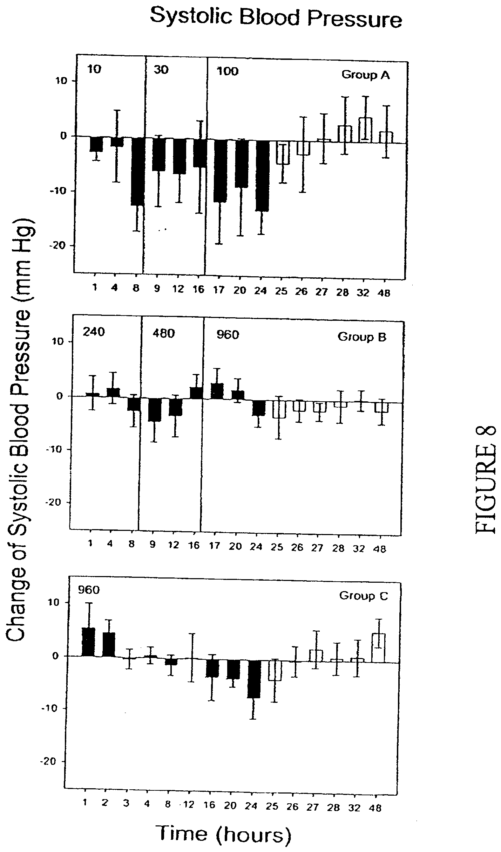

Figure 8 illustrates systolic blood pressure and relaxin. The graph shows infusion (black bars) and post-infusion (white bars) for 24 hours (either). Vertical lines mark dosage increases in Groups A and B every 8 hours. Group C received a constant dosage (all in mcg/kg/day). *, P < 0.05 vs. baseline.

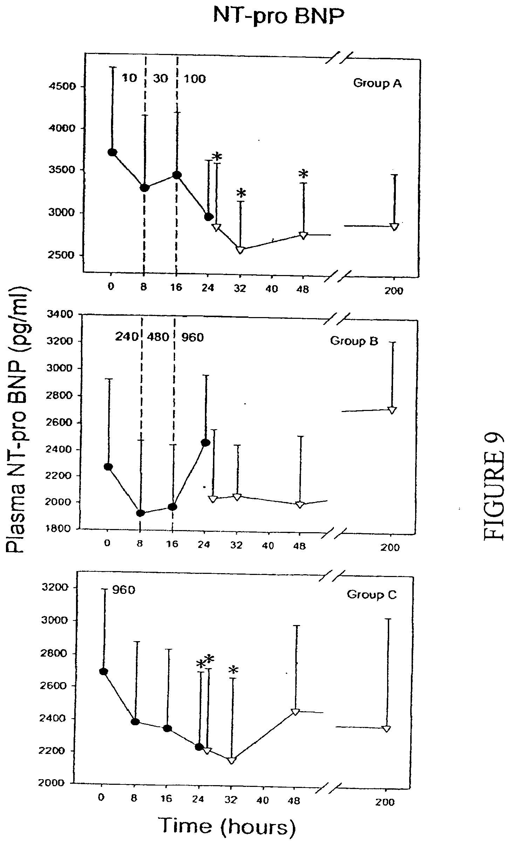

Figure 9 depicts plasma NT-pro BNP and relaxin. The graph shows infusion for 24 hours (black symbols) and post-infusion for 24 hours and Day 9 (white symbols). Vertical dash lines mark dosage increases in Groups A and B every 8 hours. Group C received a constant dosage (all in mcg/kg/day). *, P < 0.05 vs. baseline (point "0").

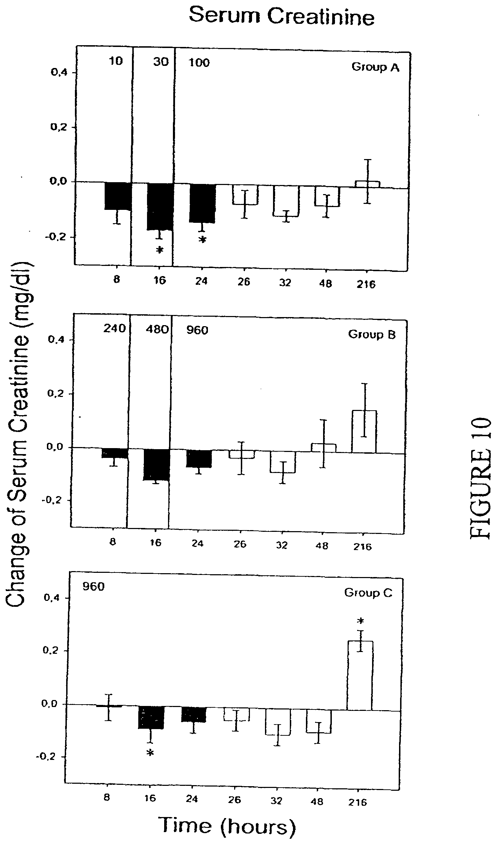

Figure 10 shows serum creatinine and relaxin. The graph shows infusion (black bars) and post-infusion (white bars) for 24 hours (either). Vertical lines mark dosage increases in Groups A and B every 8 hours. Group C received a constant dosage (all in mcg/kg/day). *, P < 0.05 vs. baseline.

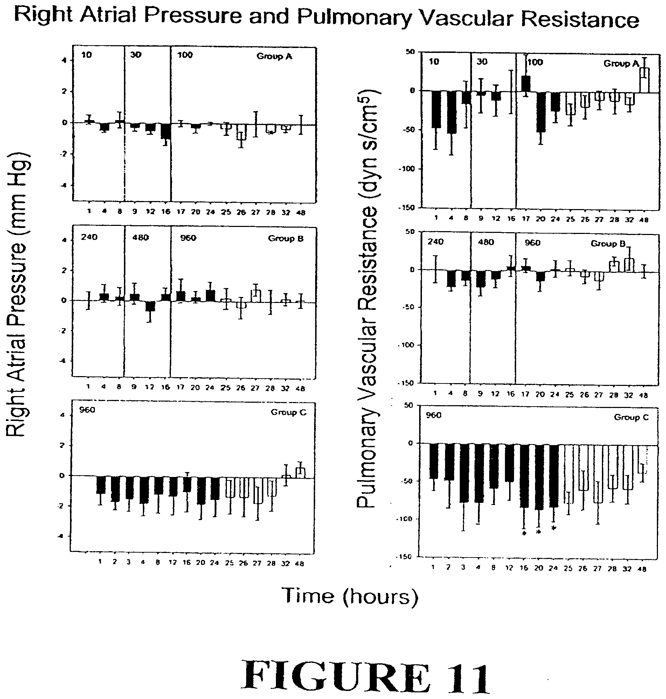

Figure 11 illustrates right atrial pressure and pulmonary vascular resistance and relaxin. The graph shows infusion (black bars) and post-infusion (white bars) for 24 hours (either). Vertical lines mark dosage increases in Groups A and B every 8 hours. Group C received a constant dosage (all in mcg/kg/day). *, P < 0.05 vs. baseline.

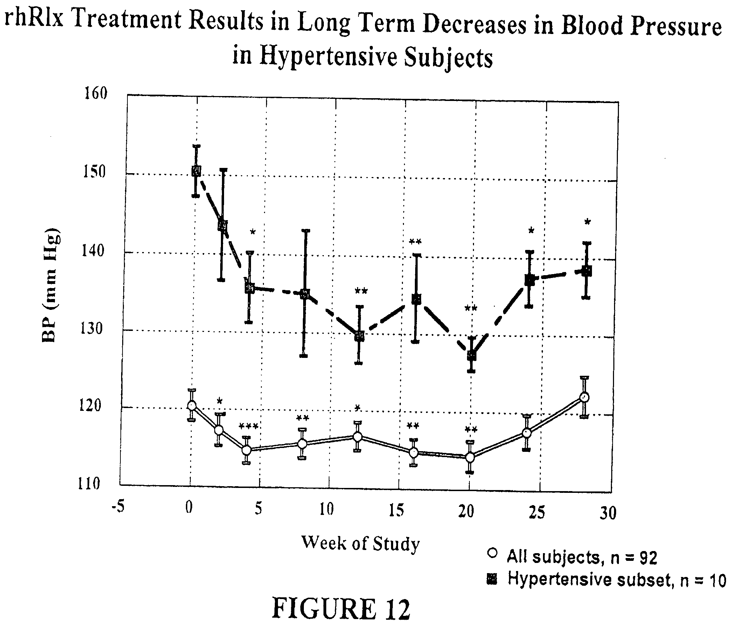

Figure 12 depicts stable decreases in systolic blood pressure (SBP) in hypertensive and normotensive subjects in the clinical trial of relaxin in patients with systemic sclerosis. Decreases in blood pressure in patients that were hypertensive at study entry was greater than the decreases in blood pressure in patients that were normotensive at study entry. Blood pressure decreases were stable during the six months of continuous dosing. None of the patients developed hypotension during dosing.

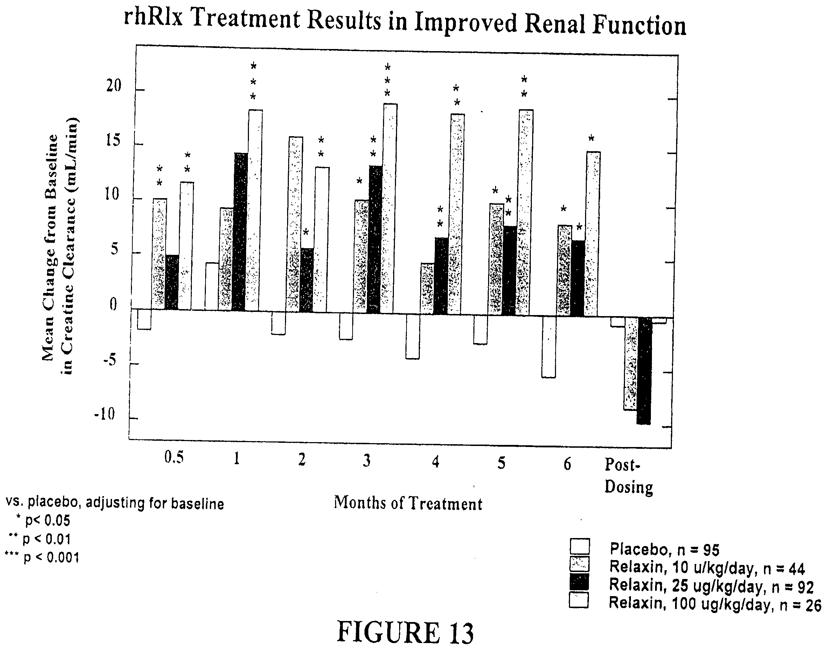

Figure 13 depicts stable improvement in renal function, measured as predicted creatinine clearance (CrCl), during six months of continuous dosing with relaxin but not with placebo in patients with systemic sclerosis.

### DETAILED DESCRIPTION

### General Overview

The present disclosure relates to methods of maintaining heart failure (HF) patients in a compensated state. Relaxin has been found to have a beneficial effect on HF patients by improving markers of renal function (e.g., decreasing blood urea nitrogen and increasing creatinine clearance), increasing the cardiac index and by decreasing systemic vascular resistance, pulmonary capillary wedge pressure, pulmonary vascular resistance, and circulating N-terminal prohormone brain natriuretic peptide. Moreover, relaxin has further advantages that have not been observed with current medications, including a reduced risk of hypotension or tachycardia during treatment. Importantly, no clinically significant adverse effects were observed from relaxin administration over the entire dose range in a pilot study described in Example 1 (Dschietzig et al., J Cardiac Fail, 15:182-90, 2009).

### Definitions

The term "relaxin" refers to a peptide hormone which is well known in the art (see Figure 1). The term "relaxin", as used herein, encompasses human relaxin, including intact full length human relaxin or a portion of the relaxin molecule that retains biological activity. The term "relaxin" encompasses human H1 preprorelaxin, prorelaxin, and relaxin; H2 preprorelaxin, prorelaxin, and relaxin; and H3 preprorelaxin, prorelaxin, and relaxin. The term "relaxin" further includes biologically active (also referred to herein as "pharmaceutically active") relaxin from recombinant, synthetic or native sources as well as relaxin variants, such as amino acid sequence variants. As such, the term contemplates synthetic human relaxin and recombinant human relaxin, including synthetic H1, H2 and H3 human relaxin and recombinant H1, H2 and H3 human relaxin. The term further encompasses active agents with relaxin-like activity, such as relaxin agonists and/or relaxin analogs and portions thereof that retain biological activity, including all agents that competitively displace bound relaxin from a relaxin receptor (*e.g*., LGR7 receptor, LGR8 receptor, GPCR135, GPCR142, *etc.*)*.* Thus, a pharmaceutically effective relaxin agonist is any agent with relaxin-like activity that is capable of binding to a relaxin receptor to elicit a relaxin-like response. In addition, the nucleic acid sequence of human relaxin as used herein must not be 100% identical to nucleic acid sequence of human relaxin (*e.g*., H1, H2 and/or H3) but may be at least about 40%, 50%, 60%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% identical to the nucleic acid sequence of human relaxin. Relaxin, as used herein, can be made by any method known to those skilled in the art. Examples of such methods are illustrated, for example, in U.S. Patent No. 5,759,807 as well as in Bullesbach et al. (1991) The Journal of Biological Chemistry 266(17):10754-10761. Examples of relaxin molecules and analogs are illustrated, for example, in U.S. Patent No. 5,166,191. Naturally occurring biologically active relaxin may be derived from human, murine *(i.e.,* rat or mouse), porcine, or other mammalian sources. Also encompassed is relaxin modified to increase *in vivo* half life, *e.g.,* PEGylated relaxin *(i.e.,* relaxin conjugated to a polyethylene glycol), modifications of amino acids in relaxin that are subject to cleavage by degrading enzymes, and the like. The term also encompasses relaxin comprising A and B chains having N- and/or C-terminal truncations. In general, in H2 relaxin, the A chain can be varied from A(1-24) to A(10-24) and B chain from B(1-33) to B(10-22); and in H1 relaxin, the A chain can be varied from A(1-24) to A(10-24) and B chain from B(1-32) to B(10-22). Also included within the scope of the term "relaxin" are other insertions, substitutions, or deletions of one or more amino acid residues, glycosylation variants, unglycosylated relaxin, organic and inorganic salts, covalently modified derivatives of relaxin, preprorelaxin, and prorelaxin. Also encompassed in the term is a relaxin analog having an amino acid sequence which differs from a wild-type (*e.g*., naturally-occurring) sequence, including, but not limited to, relaxin analogs disclosed in U.S. Pat. No. 5,811,395. Possible modifications to relaxin amino acid residues include the acetylation, formylation or similar protection of free amino groups, including the N-terminal, amidation of C-terminal groups, or the formation of esters of hydroxyl or carboxylic groups, *e.g*., modification of the tryptophan (Trp) residue at B2 by addition of a formyl group. The formyl group is a typical example of a readily-removable protecting group. Other possible modifications include replacement of one or more of the natural amino-acids in the B and/or A chains with a different amino acid (including the D-form of a natural amino-acid), including, but not limited to, replacement of the Met moiety at B24 with norleucine (Nle), valine (Val), alanine (Ala), glycine (Gly), serine (Ser), or homoserine (HomoSer). Other possible modifications include the deletion of a natural amino acid from the chain or the addition of one or more extra amino acids to the chain. Additional modifications include amino acid substitutions at the B/C and C/A junctions of prorelaxin, which modifications facilitate cleavage of the C chain from prorelaxin; and variant relaxin comprising a non-naturally occurring C peptide, *e.g.,* as described in U.S. Patent No. 5,759,807. Also encompassed by the term "relaxin" are fusion polypeptides comprising relaxin and a heterologous polypeptide. A heterologous polypeptide (*e.g*., a non-relaxin polypeptide) fusion partner may be C-terminal or N-terminal to the relaxin portion of the fusion protein. Heterologous polypeptides include immunologically detectable polypeptides (*e.g.*, "epitope tags"); polypeptides capable of generating a detectable signal (*e.g*., green fluorescent protein, enzymes such as alkaline phosphatase, and others known in the art); therapeutic polypeptides, including, but not limited to, cytokines, chemokines, and growth factors. All such variations or alterations in the structure of the relaxin molecule resulting in variants are included within the scope of this disclosure so long as the functional (biological) activity of the relaxin is maintained. Preferably, any modification of relaxin amino acid sequence or structure is one that does not increase its immunogenicity in the individual being treated with the relaxin variant. Those variants of relaxin having the described functional activity can be readily identified using *in vitro* and *in vivo* assays known in the art.

The term "heart failure" generally means that the heart is not working as efficiently as it should. Heart failure (HF) occurs when the heart muscle cannot keep up with the needs the body has for blood flow. It is a syndrome, *i.e.,* a collection of findings which may arise from a number of causes. HF can be caused by weakening of the heart muscle (*i.e.,* cardiomyopathy), leaving it unable to pump enough blood. HF is also termed congestive HF because fluids typically build up in the body, which is then said to be congested. In addition to HF caused from a weakened heart, there are also other varieties of HF. These are HFs due to the body having needs which are too high for even a normal heart to keep up with, for example, in some cases of thyroid disease in which too much thyroid hormone is produced, in patients with anemia, or several other conditions; and HF due to neurohormonal imbalances that eventually leads to acute episodes of dyspnea or other acute events such as hypertension, high blood pressure, arrhythmia, reduced renal blood flow, renal insufficiency and in severe cases mortality. If the patient has been previously diagnosed with HF, the aforementioned episodes will shift the patient from chronic HF to acute decompensated heart failure (AHF) and/or acute vascular failure. AHF will usually require hospitalization or unscheduled medical support to bring the patient fro a decompensated to a compensated state.

The terms "compensated chronic heart failure" and "compensated chronic HF" are interchangeable and describe controlled congestive heart failure generally resulting in normal cardiac output, which is generally achieved by medical intervention. Despite normal cardiac output, it is an abnormal condition in which the damaged heart maintains sufficient cardiac output by using compensatory mechanisms. As a result, compensated chronic HF is usually a progressive disease and the main goal of medical intervention is to maximize the state of stable compensated chronic HF with minimal side effects.

The terms "AHF" "acute heart failure" and "acute decompensated heart failure" as used herein is defined by the presence of all of the following at screening: dyspnea at rest or with minimal exertion, pulmonary congestion on chest X-ray and elevated natriuretic peptide levels [brain natriuretic peptide (BNP 350 pg/mL or NT-pro-BNP ≥ 1400 pg/mL].

The terms "acute cardiac decompensation" and "acute decompensation" are used interchangeably herein, and mean for the purpose of the specification and claims, an inability of the heart muscle to compensate for systemic and renal vasoconstriction due to neurohormonal imbalances in the body. Acute cardiac decompensation is characterized by altered cardiac function and fluid regulation, leading to the onset of hemodynamic instability and physiologic changes (particularly congestion and edema), and heart failure symptoms (most commonly dyspnea). This form of functional decompensation could be misdiagnosed as being caused by a valvular or myocardial defect (*i.e.,* a structural defect) although it is not usually associated with hypotension. However, "acute cardiac decompensation", as used herein, is a functional decompensation that is often associated with any one or more of certain decompensation events, including but not limited to, dyspnea, hypertension, high blood pressure, arrhythmia, reduced renal blood flow, renal insufficiency and mortality. Patients, who present with "acute cardiac decompensation", as used herein, typically have, but may not have previously been diagnosed with congestive or chronic heart failure. Such patients may have a history of heart disease or the complete absence thereof.

The term "vasculature" refers to the network of blood vessels in an organ or body part, including arteries and capillaries.

The term "balanced vasodilation" means, for purpose of the specification and claims, a dual vasodilation that occurs in the systemic (mostly arterial) and renal vasculature as a result of the binding of relaxin or a relaxin agonist to specific relaxin receptors (see detailed description, *vide infra*).

The terms "neurohormonal imbalance" and "neurohumoral imbalance" are used interchangeably herein, and refer to a hormonal disturbance in the body that can lead to heart failure. For example, excessive signaling through Gs-coupled adrenergic or Gq-coupled angiotensin pathways can cause neurohormonal imbalances. In both cases, excessive neurohormonal signaling can cause, as well as accelerate, functional decompensation (see Schrier et al., The New England Journal of Medicine 341(8):577-585, 1999). In addition, excessive neurohormonal signaling can cause, as well as accelerate, acute vascular failure.

The term "fluid overload", as used herein, refers to a condition that occurs when the blood contains too much water. Fluid overload (hypervolemia) is commonly seen with heart failure that can cause fluid overload by activation of the renin-angiotensin-aldosterone system. This fluid, primarily salt and water, builds up in various locations in the body and leads to an increase in weight, swelling in the legs and arms (peripheral edema), and/or in the abdomen (ascites). Eventually, the fluid enters the air spaces in the lungs, reduces the amount of oxygen that can enter the blood, and causes shortness of breath (dyspnea). Fluid can also collect in the lungs when lying down at night and can make night time breathing and sleeping difficult (paroxysmal nocturnal dyspnea). Fluid overload is one of the most prominent features of congestive HF.

The term "cardiac arrhythmia" means a condition where the muscle contraction of the heart becomes irregular. An unusually fast rhythm (more than 100 beats per minute) is called tachycardia. An unusually slow rhythm (fewer than 60 beats per minute) is called bradycardia.

"Cardiac ischemia" occurs when blood flow to the heart muscle (myocardium) is obstructed by a partial or complete blockage of a coronary artery. A sudden, severe blockage may lead to a heart attack (myocardial infarction). Cardiac ischemia may also cause a serious abnormal heart rhythm (arrhythmia), which can cause fainting and in severe cases death.

The term "pathophysiological" refers to a disturbance of any normal mechanical, physical, or biochemical function, either caused by a disease, or resulting from a disease or abnormal syndrome or condition that may not qualify to be called a disease. "Pathophysiology" is the study of the biological and physical manifestations of disease as they correlate with the underlying abnormalities and physiological disturbances.

The term "nitric oxide" and "NO" are used interchangeably herein and refer to an important signaling molecule involved in many physiological and pathological processes within the mammalian body, including in humans. NO can act as a vasodilator that relaxes the smooth muscle in blood vessels, which causes them to dilate. Dilation of arterial blood vessels (mainly arterioles) leads to a decrease in blood pressure. Relaxin is believed to elicit at least some vasodilation through NO. As such, relaxin binds to specific relaxin receptors such as LGR7 and LGR8 receptors on smooth muscle cells of the vasculature which in turn activates the endothelin cascade to activate nitric oxide synthase (NOS) to produce NO (see Figure 2).

The term "cardiac index" or abbreviated "CI" describes the amount of blood that the left ventricle ejects into the systemic circulation in one minute, measured in liters per minute (1/min). It is a vasodynamic parameter that relates the cardiac output (CO) to body surface area (BSA) and thus relating heart performance to the size of the individual, resulting in a value with the unit of measurement of liters per minute per square meter (1/min/m2).

The terms "AHF," "acute heart failure" and "acute decompensated heart failure" as used herein is defined by the presence of all of the following at screening: dyspnea at rest or with minimal exertion, pulmonary congestion on chest X-ray and elevated natriuretic peptide levels [brain natriuretic peptide (BNP ≥ 350 pg/mL or NT-pro-BNP ≥ 1400 pg/mL].

The term "dyspnea" refers to difficult or labored breathing. It is a sign of a variety of disorders and is primarily an indication of inadequate ventilation or of insufficient amounts of oxygen in the circulating blood. The term "orthopnea" refers to difficult or labored breathing when lying flat, which is relieved when in an upright position (sitting or standing as opposed to reclining).

Clinical studies and practice guidelines typically define hypertension as a systolic blood pressure (SBP) greater than about 140 mm Hg, and normal blood pressure as a SBP below about 140 mm Hg, 130 mm Hg or 120 mm Hg, depending upon the particular study or guideline. In the context of acute heart failure or other cardiac disease, hypotension may be characterized as a SBP below about 110 mm Hg, 100 mm Hg, or 90 mm Hg. In some preferred embodiments, the phrase a "normotensive or hypertensive state" refers to a SBP of greater than 125 mmHg at the time of study screening or relaxin administration.

As used herein, the phrase "impaired renal function" is defined as an estimated glomerular filtration rate (eGFR) of between 30 to 75 mL/min/1.73 m2, calculated using the simplified Modification of Diet in Renal Disease (sMDRD) equation.

The term "placebo" refers to a physiologically inert treatment that is often compared in clinical research trials to a physiologically active treatment. These trials are usually carried out as double blind studies and neither the prescribing doctor nor the patients know if they are taking the active drug or the substance without any apparent pharmaceutical effect (placebo). It has been observed that a patient receiving a physiologically inert treatment can demonstrate improvement for his or her condition if he or she believes they are receiving the physiologically active treatment (placebo effect). Therefore, the inclusion of a placebo in a trial assures that the statistically significant beneficial effect is related to the physiologically active treatment and not simply a result of a placebo effect.

The definition of "rehospitalization" is a hospital readmission during a certain time period after initial treatment. The time period is generally dependent on the kind of treatment and the condition of the patient.

As used herein the term "cardiovascular death" refers to death that is primarily due to a cardiovascular cause, such as death due to stroke, acute myocardial infarction, refractory congestive heart failure and any sudden.

A "loop diuretic" means a drug used in patients with congestive heart failure or renal insufficiency to reduce symptoms of hypertension and edema. A loop diuretic belongs to a class of diuretic agents that reduces readsorption of sodium and chloride by the kidney leading to an increased secretion of urine.

The term "about" when used in the context of a stated value, encompasses a range of up to 10% above or below the stated value (e.g., 90-110% of the stated value). For instance, an intravenous (IV) infusion rate of about 30 mcg/kg/day, encompasses IV infusion rates of 27 mcg/kg/day to 33 mcg/kg/day.

"Therapeutically effective" refers to the amount of pharmaceutically active relaxin that will result in a measurable desired medical or clinical benefit to a patient, as compared to the patient's baseline status or to the status of an untreated or placebo-treated (e.g., not treated with relaxin) subject.

### Relaxin

Relaxin is a peptide hormone that is similar in size and shape to insulin (see Figure 1). More specifically, relaxin is an endocrine and autocrine/paracrine hormone which belongs to the insulin gene superfamily. The active form of the encoded protein consists of an A chain and a B chain, held together by disulphide bonds, two inter-chains and one intra-chain. Thus, the structure closely resembles insulin in the disposition of disulphide bonds. In humans, there are three known non-allelic relaxin genes, relaxin-1 (RLN-1 or H1), relaxin-2 (RLN-2 or H2) and relaxin-3 (RLN-3 or H3). H1 and H2 share high sequence homology. There are two alternatively spliced transcript variants encoding different isoforms described for this gene. H1 and H2 are differentially expressed in reproductive organs (see U.S. Patent No. 5,023,321 and Garibay-Tupas et al. (2004) Molecular and Cellular Endocrinology 219:115-125) while H3 is found primarily in the brain. The evolution of the relaxin peptide family in its receptors is generally well known in the art (see Wilkinson et al. (2005) BMC Evolutionary Biology 5(14):1-17; and Wilkinson and Bathgate (2007) Chapter 1, Relaxin and Related Peptides, Landes Bioscience and Springer Science + Business Media).

Relaxin activates specific relaxin receptors, *i.e.,* LGR7 (RXFP1) and LGR8 (RXFP2) as well as GPCR135 and GPCR142. LGR7 and LGR8 are leucine-rich repeat-containing, G protein-coupled receptors (LGRs) which represent a unique subgroup of G protein-coupled receptors. They contain a heptahelical transmembrane domain and a large glycosylated ectodomain, distantly related to the receptors for the glycoproteohormones, such as the LH-receptor or FSH-receptor. These relaxin receptors are found in the heart, smooth muscle, connective tissue, and central and autonomous nervous system. Potent relaxins such as H1, H2, porcine and whale relaxin possess a certain sequence in common, *i.e.,* the Arg-Glu-Leu-Val-Arg-X-X-Ile sequence or binding cassette. These relaxins activate the LGR7 and LGR8 receptors. Relaxins that deviate from his sequence homology such as rat, shark, dog and horse relaxins show a reduction in bioactivity through the LGR7 and LGR8 receptors (see Bathgate et al. (2005) Ann. N.Y. Acad Sci. 1041:61-76; *Receptors for Relaxin Family Peptides*). However, similar to H2 relaxin, H3 relaxin activates the LGR7 receptor (see Satoko et al. (2003) The Journal of Biological Chemistry 278(10):7855-7862). In addition, H3 has been shown to activate the GPCR135 receptor (see Van der Westhuizen (2005) Ann. N.Y. Acad. Sci. 1041:332-337) and GPCR142 receptor. GPCR135 and GPCR142 are two structurally related G-protein-coupled receptors. Mouse and rat GPCR135 exhibit high homology (*i.e.,* greater than 85%) to the human GPCR135 and have very similar pharmacological properties to that of the human GPCR135. Human and mouse as well as rat relaxin-3 binds to and activates mouse, rat, and human GPCR135 at high affinity. In contrast, the mouse GPCR142 is less well conserved *(i.e.,* 74% homology) with human GPCR142. GPCR142 genes from monkey, cow, and pig were cloned and shown to be highly homologous *(i.e.,* greater than 84%) to human GPCR142. Pharmacological characterization of GPCR142 from different species has shown that relaxin-3 binds to GPCR142 from different species at high affinity (see Chen et al. (2005) The Journal of Pharmacology and Experimental Therapeutics 312(1):83-95).

Relaxin is found in both, women and men (see Tregear et al.; Relaxin 2000, Proceedings of the Third International Conference on Relaxin & Related Peptides (22-27 October 2000, Broome, Australia). In women, relaxin is produced by the *corpus luteum* of the ovary, the breast and, during pregnancy, also by the placenta, chorion, and decidua. In men, relaxin is produced in the testes. Relaxin levels rise after ovulation as a result of its production by the *corpus luteum* and its peak is reached during the first trimester, not toward the end of pregnancy. In the absence of pregnancy its level declines. In humans, relaxin is plays a role in pregnancy, in enhancing sperm motility, regulating blood pressure, controlling heart rate and releasing oxytocin and vasopressin. In animals, relaxin widens the pubic bone, facilitates labor, softens the cervix (cervical ripening), and relaxes the uterine musculature. In animals, relaxin also affects collagen metabolism, inhibiting collagen synthesis and enhancing its breakdown by increasing matrix metalloproteinases. It also enhances angiogenesis and is a renal vasodilator.

Relaxin has the general properties of a growth factor and is capable of altering the nature of connective tissue and influencing smooth muscle contraction. H1 and H2 are believed to be primarily expressed in reproductive tissue while H3 is known to be primarily expressed in brain (*supra*)*.* However, as determined during development of the present disclosure H2 and H3 play a major role in cardiovascular and cardiorenal function and can thus be used to treat associated diseases. H1 can be employed similarly due to its homology with H2. In addition, pharmaceutically effective relaxin agonists with relaxin-like activity would be capable of activating relaxin receptors to elicit a relaxin-like response.

### Relaxin Agonists

In some embodiments, the present disclosure provides methods of treating patients diagnosed with chronic heart failure comprising administration of a relaxin agonist. In some methods, the relaxin agonist activates one or more relaxin-related G-protein coupled receptors (GPCR) selected from but not limited to RXFP1, RXFP2, RXFP3, RXFP4, FSHR (LGR1), LHCGR (LGR2), TSHR (LGR3), LGR4, LGR5, LGR6 LGR7 (RXFP1) and LGR8 (RXFP2). In some embodiments, the relaxin agonist comprises the amino acid sequence of Formula I of WO 2009/007848 of Compugen (herein incorporated by reference for the teaching of relaxin agonist sequences).

Formula I peptides are preferably from 7 to 100 amino acids in length and comprise the amino acid sequence: X1- X2- X3- X4- X5- X6- X7- X8- X9- X10- X1 1- X12- X13-X14- X15- X16- X17- X18- X19- X20-X21- X22- X23- X24- X25- X26- X27- X28- X29-X30- X31- X32- X33; wherein X1 is absent or G or a small naturally or non-naturally occurring amino acid; X2 is absent or Q or a polar naturally or non-naturally occurring amino acid; X3 is absent or K or a basic naturally or non-naturally occurring amino acid; X4 is absent or G or a small naturally or non-naturally occurring amino acid; X5 is absent or Q or S a polar naturally or non-naturally occurring amino acid; X6 is absent or V or A or P or M or a hydrophobic naturally or non-naturally occurring amino acid; X7 is absent or G or a small naturally or non-naturally occurring amino acid; X8 is absent or P or L or A naturally or non-naturally occurring amino acid; X9 is absent or P or Q naturally or non-naturally occurring amino acid; X10 is absent or G or a small naturally or non-naturally occurring amino acid; X11 is absent or A or H or E or D or a hydrophobic or a small or an acidic naturally or non-naturally occurring amino acid; X12 is absent or A or P or Q or S or R or H or a hydrophobic or a small naturally or non- naturally occurring amino acid; X13 is absent or C or V or a hydrophobic naturally or non-naturally occurring amino acid; X14 is absent or R or K or Q or P or a basic or a polar naturally or non-naturally occurring amino acid; X15 is absent or R or Q or S or a basic or a polar naturally or non-naturally occurring amino acid; X16 is absent or A or L or H or Q or a hydrophobic or a small naturally or non-naturally occurring amino acid; X17 is absent or Y or a hydrophobic or an aromatic naturally or non-naturally occurring amino acid; X18 is absent or A or a hydrophobic or small naturally or non-naturally occurring amino acid; X19 is absent or A or a hydrophobic small naturally or non-naturally occurring amino acid; X20 is absent or F or a hydrophobic or an aromatic naturally or non-naturally occurring amino acid; X21 is absent or S or T or a polar naturally or non-naturally occurring amino acid; X22 is absent or V or a hydrophobic naturally or non-naturally occurring amino acid; X23 is absent or G or hydrophobic or small non-naturally occurring amino acid or replaced by an amide; X24 is absent or R or a basic naturally or non-naturally occurring amino acid; X25 is absent or R or a basic naturally or non-naturally occurring amino acid; X26 is A or a hydrophobic or small naturally or non-naturally occurring amino acid; X27 is Y or a hydrophobic or an aromatic naturally or non-naturally occurring amino acid; X28 is A or a hydrophobic or small naturally or non-naturally occurring amino acid; X29 is A or a hydrophobic or small naturally or non-naturally occurring amino acid; X30 is F or a hydrophobic naturally or non-naturally occurring amino acid; X31 is S or T or a polar naturally or non-naturally occurring amino acid; X32 is V or a hydrophobic naturally or non-naturally occurring amino acid; X33 is absent or G or hydrophobic or small naturally or non-naturally occurring amino acid or replaced by an amide; or a pharmaceutically acceptable salt thereof (SEQ ID NO:4). In some preferred embodiments, the relaxin agonist comprises the sequence of peptide P59C13V (free acid) GQKGQVGPPGAA VRRA Y AAFSV (SEQ ID NO:5). In another preferred embodiment, the relaxin agonist comprises the sequence of peptide P74C13V (free acid) GQKGQVGPPGAA VRRA Y AAFS VGRRA Y AAFS V (SEQ DD NO: 6). Further derivatives of the human complement C1Q tumor necrosis factor-related protein 8 (CTRP8 or C1QT8) such as peptide P59-G (free acid Gly) GQKGQVGPPGAACRRA Y AAFSVG (SEQ ID NO:7) are also contemplated to be suitable for use in the methods of the present disclosure. The amino acid sequence of C 1 QT8 is set forth as SEQ ID NO:8

MAAPALLLLALLLPVGAWPGLPRRPCVHCCRPAWPPGPYARVSDRDLWRGDLWRG
LPRVRPTIDIEILKGEKGEAGVRGRAGRSGKEGPPGARGLQGRRGQKGQVGPPGAAC
RRAYAAFSVGRRAYAAFSVGRREGLHSSDHFQAVPFDTELVNLDGAFDLAAGRFLC
TVPGVYFLSLNVHTWNYKETYLHIMLNRRPAAVLYAQPSERSVMQAQSLMLLLAA
GDAVWVRMF QRDRDNAIYGEHGDLYITFSGHLVKP AAEL.

The present disclosure also encompasses homologues of these polypeptides, such homologues can be at least 50%, at least 55%, at least 60%, at least 65%, at least 70%, at least 75%, at least 80%, at least 85%, at least 85%, at least 90%, at least 95% or more say 100% identical to the amino acid sequence of an exemplary relaxin agonist (e.g., SEQ ID NO:5 or SEQ ID NO:6), as can be determined using BlastP software of the National Center of Biotechnology Information (NCBI) using default parameters, optionally and preferably including the following: filtering on (this option filters repetitive or low- complexity sequences from the query using the Seg (protein) program), scoring matrix is BLOSUM62 for proteins, word size is 3, E value is 10, gap costs are 1 1,1 (initialization and (initialization and extension). Optionally and preferably, nucleic acid sequence identity/homology is determined with BlastN software of the National Center of Biotechnology Information (NCBI) using default parameters, which preferably include using the DUST filter program, and also preferably include having an E value of 10, filtering low complexity sequences and a word size of 1 1. Finally the present disclosure also encompasses fragments of the above described polypeptides and polypeptides having mutations, such as deletions, insertions or substitutions of one or more amino acids, either naturally occurring or artificially induced, either randomly or in a targeted fashion.

### Methods of Treatment

### A. Increase in Cardiac Index (CI)

For New York Hear Association (NYHA) classification Class II and Class III patients living with chronic compensated heart failure, restrictions in everyday quality of life do exist despite optimal standard drug therapy. The cardiac index (CI) and cardiac output (CO) is reduced in most patients with chronic HF. Since the heart in these patients is not performing optimally drugs are used to compensate for the impairment of the heart. However, current drugs have side effects such as hypotension and renal toxicity. In contrast, relaxin treatment increases the CI and CO in chronic compensated HF patients without deleterious side effects. In particular, relaxin administration does not increase a subject's heart rate, but reduce systemic vascular resistance without causing hypotension, or worsening renal function. The most common cause of heart failure is left ventricular systolic dysfunction resulting in reduced cardiac contractility leading to a low CI and high pulmonary pressures. Importantly, in addition to increasing the CI, relaxin has been demonstrated to decrease the pulmonary capillary wedge pressure in chronic heart failure patients. Thus, relaxin has many characteristics that may be highly beneficial for chronic HF patients, such as those with a below normal CI. In addition, the therapeutically effective amount of relaxin can administered at a fixed dose without the need for prior titration. That dose is usually between about 10 and 960 mcg/kg/day. However, relaxin can also be administered to chronic compensated heart failure patients at a fixed dose of 960 mcg/kg/day. This dose has been shown to result in a significant increase of the CI and a reduction of pulmonary capillary wedge pressure. Relaxin may be administered intravenously for 8 or 24, or up to 48 hours, or for as long as needed (e.g. 7, 14, 21 days etc.). However, additional routes and schedules of delivery are also suitable and some of these have been described in more detail in the "Administration and Dosing Regimen" section of this disclosure. Beside these benefits for treating patients that already underwent left ventricular remodeling of the heart, it is obviously most important to prevent such remodeling to slow the progression of heart failure disease. Thus, relaxin may be beneficial for a population who do not responding to standard preventive measures, in order to avoid further development of structural heart disease.

### B. Improve Functional Capacity

Chronic HF patients with more symptomatic or advanced disease, such as NYHA classification Class III and especially Class IV can generally only be sub-optimally managed. Symptoms, often significant, persist despite treatment, and development of drug tolerance or side effects can further reduce the therapeutic success of current treatments. To improve quality of life in patients with advanced heart failure or with refractory symptoms of HF at rest, intervention with relaxin may be beneficial. Current intravenously administered inotropes and vasodilators such as dobutamine, milrinone, nitroglycerin, nitroprusside, or nesiritide, used to treat HF patients with severe symptoms, including refractory symptoms at rest, have restrictions that limit their use for chronic compensated HF patients (e.g., limited effectiveness, renal toxicity, risk of hypotension, or titration requirements). However, the therapeutically effective amount of relaxin can administered at a fixed dose without the need for prior titration. Furthermore, renal toxicity with relaxin has not been observed in patients over a wide dose range. Moreover, relaxin increases CI and CO in chronic compensated HF patients without increasing the heart rate and at the same time reducing systemic vascular resistance without causing hypotension, or worsening renal function. Importantly, in addition to increasing the CI, relaxin has been demonstrated to decrease the pulmonary capillary wedge pressure in chronic heart failure patients. Thus, relaxin has many characteristics that may be highly beneficial for chronic HF patients, including patients with advanced disease. The administered dose is usually between about 10 and 960 mcg/kg/day. However, relaxin can also be administered in chronic compensated heart failure patients at a fixed dose of 960 mcg/kg/day. This dose has been shown to result in a significant increase in CI and a reduction of pulmonary capillary wedge pressure. Relaxin may be administered intravenously for 8 or 24, or up to 48 hours, or for as long as needed (e.g. 7, 14, 21 days etc.). However, additional routes and schedules of delivery are also suitable and some of these have been described in more detail in the "Administration and Dosing Regimen" section of this disclosure.

### C. Reduce Frequency of Decompensation Episodes

Administration of relaxin in patients with stable compensated chronic HF (e.g., patients in which compensation has been achieved by an established drug treatment regimen), results in an even further beneficial outcome with an increased cardiac index, and a decrease in systemic vascular resistance, pulmonary capillary wedge pressure, pulmonary vascular resistance, circulating N-terminal prohormone brain natriuretic peptide, and markers of renal dysfunction, (blood urea nitrogen and creatinine). Importantly, simultaneously with these benefits of relaxin, there is no significant risk of hypotension, tachycardia and/or arrhythmia as a result of relaxin treatment. As such, relaxin can provide a stabilizing and salubrious effect to the stable compensated chronic HF population, resulting in a lower risk of decompensation and and a reduced frequency of decompensation episodes requiring hospitalization. Thus, these distinctive characteristics of relaxin are indicative of a beneficial use of relaxin for outpatients with manageable chronic HF diagnosed with Class II, or Class III heart failure according to NYHA classification. The administered dose is usually between about 10 and 960 mcg/kg/day. Relaxin may be administered intravenously for 8 or 24, or up to 48 hours, or for as long as needed (e.g. 7, 14, 21 days etc.). However, additional routes and schedules of delivery are also suitable and some of these have been described in more detail in the "Administration and Dosing Regimen" section of this disclosure.

### D. Reduce Use of Concurrent Chronic Heart Failure Medications

There are a wide variety of approved drugs currently in use to manage patients with chronic HF. Patients at risk for heart failure are treated to control underlying causes such as hypertension, and lipid disorders. Certain patients with diabetes and vascular disorders receive medications such as vasodilators, adrenergic blockers, centrally acting alpha-agonists, angiotensin-converting enzyme (ACE) inhibitors, angiotensin II receptor blockers (ARBs), calcium channel blockers, positive inotropes, and multiple types of diuretics (e.g., loop, potassium-sparing, thiazide and thiazide-like). In some embodiments, the present disclosure provides methods of treating heart failure comprising administration of relaxin in combination with an adjunct therapy such as an antihypertensive drug. In some methods, the antihypertensive drug is selected from but not limited to an anti-platelet, a beta-blocker, a diuretic, and an anti-angiotensin therapy.

Angiotensin Converting Enzyme (ACE) inhibitors have been used for the treatment of hypertension for many years. ACE inhibitors block the formation of angiotensin II, a hormone with adverse effects on the heart and circulation in CHF patients. Side effects of these drugs include a dry cough, low blood pressure, worsening kidney function and electrolyte imbalances, and sometimes, allergic reactions. Examples of ACE inhibitors include captopril (CAPOTEN), enalapril (VASOTEC), lisinopril (ZESTRIL, PRINIVIL), benazepril (LOTENSIN), and ramipril (ALTACE). For those patients who are unable to tolerate ACE inhibitors, an alternative group of drugs, called the angiotensin receptor blockers (ARBs), can be used. These drugs act on the same hormonal pathway as ACE inhibitors, but instead block the action of angiotensin II at its receptor site directly. Side effects of these drugs are similar to those associated with ACE inhibitors, although the dry cough is less common. Examples of this class of medications include losartan (COZAAR), candesartan (ATACAND), telmisartan (MICARDIS), valsartan (DIOVAN), and irbesartan (AVAPRO).

Beta-blockers are drugs that block the action of certain stimulating hormones, such as epinephrine (adrenaline), norepinephrine, and other similar hormones, which act on the beta receptors of various body tissues. The natural effect of these hormones on the beta receptors of the heart is a more forceful contraction of the heart muscle. Beta-blockers are agents that block the action of these stimulating hormones on the beta receptors. The stimulating effect of these hormones, while initially useful in maintaining heart function, appears to have detrimental effects on the heart muscle over time. Generally, if chronic HF patients receive beta-blockers they are given at a very low dose at first which is then gradually increased. Side effects include fluid retention, low blood pressure, low pulse, and general fatigue and lightheadedness. Beta-blockers should also not be used in people with diseases of the airways (e.g., asthma, emphysema) or very low resting heart rates. Carvedilol (COREG) has been the most thoroughly studied drug in the setting of congestive heart failure and remains the only beta-blocker with FDA approval for the treatment of congestive heart failure. However, research comparing carvedilol directly with other beta-blockers in the treatment of congestive heart failure is ongoing. Long acting metopropol (TOPROL XL) is also effective in patients with congestive heart failure. Digoxin (LANOXIN) is naturally produced by the Foxglove flowering plant and has been used for treatment of chronic HF patients for a decade. Digoxin stimulates the heart muscle to contract more forcefully. Side effects include nausea, vomiting, heart rhythm disturbances, kidney dysfunction, and electrolyte abnormalities. In patients with significant kidney impairment the dose of digoxin needs to be carefully adjusted and monitored.

Diuretics are often used in the treatment of chronic HF patients to prevent or alleviate the symptoms of fluid retention. These drugs help keep fluid from building up in the lungs and other tissues by promoting the flow of fluid through the kidneys. Although they are effective in relieving symptoms such as shortness of breath and leg swelling, they have not been demonstrated to positively impact long term survival. When hospitalization is required, diuretics are often administered intravenously because the ability to absorb oral diuretics may be impaired. Side effects of diuretics include dehydration, electrolyte abnormalities, particularly low potassium levels, hearing disturbances, and low blood pressure. It is important to prevent low potassium levels by providing supplements to patients, when appropriate. Any electrolyte imbalances may make patients susceptible to serious heart rhythm disturbances. Examples of various classes of diuretics include furosemide (LASIX), hydrochlorothiazide, bumetanide (BUMEX), torsemide (DEMADEX), and metolazone (ZAROXOLYN). Spironolactone (ALDACTONE) has been used for many years as a relatively weak diuretic in the treatment of various diseases. This drug blocks the action of the hormone aldosterone. Aldosterone has theoretical detrimental effects on the heart and circulation in congestive heart failure. Its release is stimulated in part by angiotensin II (supra). Side effects of this drug include elevated potassium levels and, in males, breast tissue growth (gynecomastia). Another aldosterone inhibitor is eplerenone (INSPRA).

The beneficial characteristics of relaxin that have been observed in treated patients, such as an increased cardiac index and a decrease in systemic vascular resistance, pulmonary capillary wedge pressure, pulmonary vascular resistance, circulating N-terminal prohormone brain natriuretic peptide, and markers of renal dysfunction (blood urea nitrogen and creatinine), indicate that it is desirable to administer relaxin instead of or in addition to currently approved heart failure drugs. Relaxin has many advantages that have not been observed with current medications, including no significant risk of hypotension and tachycardia during treatment, no need for titration prior administration, and no renal toxicity. HF patients under standard drug treatment to achieve and maintain a state of stable compensated HF can receive relaxin administered at a dose that is generally between about 10 and 960 mcg/kg/day. Relaxin may be administered intravenously for 8 or 24, or up to 48 hours, or for as long as needed (e.g. 7, 14, 21 days etc.). However, additional routes and schedules of delivery are also suitable and some of these have been described in more detail in the "Administration and Dosing Regimen" section of this disclosure. A beneficial effect of relaxin is even found in chronic compensated HF patients when relaxin is administered in addition to optimal standard therapy. The outcome confirms the benefits described above for relaxin and indicates a reduction in dose or discontinuation of one or more concurrent HF medications.

### E. Additional Treatment Methods

*Relaxin Treatment Results in Balanced Vasodilation.* The beneficial effect of relaxin is believed to be a direct result of relaxin acting as a receptor-specific vasodilator in the renal and systemic vasculature by binding to specific relaxin receptors that are found on the smooth muscle tissue of the vasculature. This in turn results in balanced vasodilation as both systemic and renal arteries are vasodilated in a moderate but effective way without causing hypotension in the treated patient. This property of relaxin as a receptor-specific and balancing vasodilator is particularly advantageous in context in which it is desirable to obtain increased vasodilation in specific areas of the body where vasoconstriction causes a serious ill effect such as in the arteries that supply blood to the heart and the kidneys. Notably, the balanced vasodilation occurs without causing any deleterious side effect during the process of treatment. A common problem with treatment of non-specific vasodilators is that these drugs often lead to serious side effects in the treated patient, mainly because general agonists act too potently and non-specifically. In comparison, the moderate effect of relaxin slowly increases vasodilation in areas of the body where it is needed the most. It is important to note that relaxin treatment does not cause hypotension as is the case with many drugs which overcompensate for vasoconstriction. In particular, non-specific vasodilators can cause large and small arteries and veins throughout the body to dilate excessively leading to hypotension. Thus, when the patient receives a pharmaceutical composition with pharmaceutically active relaxin or pharmaceutically effective relaxin agonist which targets systemic and renal blood vessels via localized specific relaxin receptors (e.g., LRG7, LGR8, GPCR135, GPCR142 receptors) the result is balanced vasodilation without hypotension.

Furthermore, as determined during development of the present disclosure, balanced vasodilation in heart failure patients caused by relaxin is a form of dual vasodilation of systemic (mostly arterial) and renal vasculature. Relaxin, however, causes the vasodilation in patients with heart failure to be balanced because relaxin adds an actual renal vasodilation to the systemic vasodilation and, thus, achieves a balance between the systemic and renal vasculature. Previous drugs are known to cause some indirect renal improvement as a result of systemic vasodilation but not enough to achieve this balance. In fact, the vasodilative balance caused by relaxin administration allows the AHF patient to move from an acute state to a stable state in a relatively short period of time. In addition, administration of relaxin in patients with stable compensated chronic HF, achieved by current established drug treatment, results in even further beneficial outcome with decreased markers of renal dysfunction and advantageous hemodynamic effects consistent with vasodilation. As such, relaxin can provide a stabilizing and salubrious effect to the stable compensated chronic HF population, resulting in a lower risk of decompensation and possibly slowing down the progression of this disease, resulting in a reduced frequency of decompensation episodes requiring hospitalization in compensated chronic heart failure patients. Moreover, an increase in cardiac index without an increase in heart rate together with the decrease of other parameters such as systemic vascular resistance, pulmonary capillary wedge pressure, pulmonary vascular resistance, blood urea nitrogen, creatinine, and circulating N-terminal prohormone brain natriuretic peptide, is indicative of a beneficial use of relaxin for both chronic HF patients in general as well as for advanced HF patients in need of a more aggressive therapy regimen.

These beneficial effects of relaxin involve the binding of relaxin to its receptors (*e.g.*, LRG7, LGR8, GPCR135, GPCR142 receptors) resulting in balanced vasodilation, *i.e.,* a dual vasodilation in both the systemic and renal vasculature. Consequently, relaxin can be used to reduce the risk of cardiac decompensation events or by limiting progression of the disease by selecting human subjects with stable compensated heart failure and administering to those subjects a pharmaceutical formulation with pharmaceutically active relaxin. Particularly, such subjects receive pharmaceutically active human relaxin *(e.g.,* synthetic, recombinant) or pharmaceutically effective relaxin agonist in an amount in a range of about 10 to 960 mcg/kg of subject body weight per day. Relaxin may be administered intravenously for 8 or 24, or up to 48 hours, or for as long as needed (e.g. 7, 14, 21 days etc.). However, additional routes and schedules of delivery are also suitable and some of these have been described in more detail in the "Administration and Dosing Regimen" section of this disclosure. The administration of relaxin is continued as to maintain a serum concentration of relaxin from about 0.5 to about 500 ng/ml, more preferably from about 3 to about 300 ng/ml. Thus, the methods of the present disclosure include administrations that result in these serum concentrations of relaxin. These relaxin concentrations can reduce or prevent the progression of the disease and the risk of having decompensation events such as dyspnea, hypertension, arrhythmia, reduced renal blood flow, and renal insufficiency.

*Relaxin Treatment Is Not Associated With Renal Toxicity.* Renal dysfunction is a common and progressive complication of chronic HF. The clinical course typically fluctuates with the patient's clinical status and treatment. Despite the growing recognition of the frequent presentation of combined cardiac and renal dysfunction, also termed the "cardiorenal syndrome," its underlying pathophysiology is not well understood. No consensus as to its appropriate management has been achieved in the art. Because patients with chronic heart failure are surviving longer and die less frequently from cardiac arrhythmia, cardiorenal syndrome is more and more prevalent and proper management is needed (Gary Francis (2006) Cleveland Clinic Journal of Medicine 73(2):1-13). Relaxin is administered to the subject and performs a dual action by binding to the relaxin receptors in the systemic and renal vasculature, resulting in balanced vasodilation. As noted above (*supra*), such subjects receive pharmaceutically active human relaxin (*e.g*., synthetic, recombinant) or pharmaceutically effective relaxin agonist in an amount in a range of about 10 to 960 mcg/kg of subject body weight per day. These dosages result in serum concentrations of relaxin of about 75, 150, and 300 ng/ml, respectively. The administration of relaxin is continued as to maintain a serum concentration of relaxin from about 0.5 to about 500 ng/ml, more preferably from about 3 to about 300 ng/ml. Relaxin may be administered intravenously for 8 or 24, or up to 48 hours, or for as long as needed (e.g. 7, 14, 21 days etc.). However, additional routes and schedules of delivery are also suitable and some of these have been described in more detail in the "Administration and Dosing Regimen" section of this disclosure.

Subjects who suffer from renal insufficiency associated with heart failure often also experience elevated levels of brain natriuretic peptide (BNP). BNP is synthesized in the cardiac ventricles in response to heart failure and left ventricular dysfunction and is used as a diagnostic marker of heart failure. Its effects include systemic vasodilation and unbalanced vasodilation in the kidney, *i.e.,* efferent arteriolar constriction and afferent arteriole vasodilation. BNP levels are even further reduced when relaxin is administered to stable compensated chronic HF patients. This makes BNP a convenient marker since it is reduced as the severity of decompensation is reduced and monitoring BNP levels in patients that are treated with relaxin is, thus, a convenient way to assure that compensated chronic HF is stabilized.

Relaxin causes low to no renal toxicity when it is given to stable compensated chronic HF patients. This means that the renal function in the patients improves rather than deteriorates as a result of treatment. Even with higher serum concentrations of about 75ng/ml relaxin is far less toxic than currently available medications (*e.g*., loop diuretics such as furosemide, angiotensin converting enzyme inhibitors such as captopril, angiotensin receptor blockers such as candesartan, and the like). One important feature of this disclosure is that relaxin preserves the renal function while causing little to no renal toxicity during treatment. Although existing drugs may preserve some of the renal function they also increase the renal toxicity in patients. This renal toxicity then further deteriorates the heart condition. In comparison, relaxin administration achieves a steady-state maintenance of most patients due in part to the absence of renal toxicity. This allows the more stable chronic HF population to achieve a manageable condition where the likelihood of exacerbating heart failure is measurably reduced.

### Relaxin Compositions and Formulations

Relaxin, relaxin agonists and/or relaxin analogs are formulated as pharmaceuticals to be used in the methods of the disclosure. Any composition or compound that can stimulate a biological response associated with the binding of biologically or pharmaceutically active relaxin (*e.g.,* synthetic relaxin, recombinant relaxin) or a relaxin agonist (*e.g.,* relaxin analog or relaxin-like modulator) to relaxin receptors can be used as a pharmaceutical in the disclosure. General details on techniques for formulation and administration are well described in the scientific literature (see Remington's Pharmaceutical Sciences, Maack Publishing Co, Easton Pa.). Pharmaceutical formulations containing pharmaceutically active relaxin can be prepared according to any method known in the art for the manufacture of pharmaceuticals. The formulations containing pharmaceutically active relaxin or relaxin agonists used in the methods of the disclosure can be formulated for administration in any conventionally acceptable way including, but not limited to, intravenously, subcutaneously, intramuscularly, sublingually, topically, orally, via inhalation, and wearable infusion pump. Illustrative examples are set forth below. In one preferred embodiment, relaxin is administered intravenously (IV).

When the drugs are delivered by intravenous injection, the formulations containing pharmaceutically active relaxin or a pharmaceutically effective relaxin agonist can be in the form of a sterile injectable preparation, such as a sterile injectable aqueous or oleaginous suspension. This suspension can be formulated according to the known art using those suitable dispersing or wetting agents and suspending agents which have been mentioned above. The sterile injectable preparation can also be a sterile injectable solution or suspension in a nontoxic parenterally-acceptable diluent or solvent. Among the acceptable vehicles and solvents that can be employed are water and Ringer's solution, an isotonic sodium chloride. In addition, sterile fixed oils can conventionally be employed as a solvent or suspending medium. For this purpose any bland fixed oil can be employed including synthetic mono- or diglycerides. In addition, fatty acids such as oleic acid can likewise be used in the preparation of injectables.

Pharmaceutical formulations for oral administration can be formulated using pharmaceutically acceptable carriers well known in the art in dosages suitable for oral administration. Such carriers enable the pharmaceutical formulations to be formulated in unit dosage forms as tablets, pills, powder, capsules, liquids, lozenges, gels, syrups, slurries, suspensions, *etc.,* suitable for ingestion by the patient. Pharmaceutical preparations for oral use can be obtained through combination of relaxin compounds with a solid excipient, optionally grinding a resulting mixture, and processing the mixture of granules, after adding suitable additional compounds, if desired, to obtain tablets or pills. Suitable solid excipients are carbohydrate or protein fillers which include, but are not limited to, sugars, including lactose, sucrose, mannitol, or sorbitol; starch from corn, wheat, rice, potato, or other plants; cellulose such as methyl cellulose, hydroxypropylmethyl-cellulose, or sodium carboxymethylcellulose; and gums including arabic and tragacanth; as well as proteins such as gelatin and collagen. If desired, disintegrating or solubilizing agents may be added, such as the cross-linked polyvinyl pyrrolidone, agar, alginic acid, or a salt thereof, such as sodium alginate. Pharmaceutical preparations of the disclosure that can also be used orally are, for example, push-fit capsules made of gelatin, as well as soft, sealed capsules made of gelatin and a coating such as glycerol or sorbitol. Push-fit capsules can contain relaxin mixed with a filler or binders such as lactose or starches, lubricants such as talc or magnesium stearate, and, optionally, stabilizers. In soft capsules, the relaxin compounds may be dissolved or suspended in suitable liquids, such as fatty oils, liquid paraffin, or liquid polyethylene glycol with or without stabilizers.

Aqueous suspensions of the disclosure contain relaxin in admixture with excipients suitable for the manufacture of aqueous suspensions. Such excipients include a suspending agent, such as sodium carboxymethylcellulose, methylcellulose, hydroxypropylnethylcellulose, sodium alginate, polyvinylpyrrolidone, gum tragacanth and gum acacia, and dispersing or wetting agents such as a naturally occurring phosphatide (*e.g*., lecithin), a condensation product of an alkylene oxide with a fatty acid (*e.g*., polyoxyethylene stearate), a condensation product of ethylene oxide with a long chain aliphatic alcohol (*e.g.,* heptadecaethylene oxycetanol), a condensation product of ethylene oxide with a partial ester derived from a fatty acid and a hexitol (*e.g*., polyoxyethylene sorbitol mono-oleate), or a condensation product of ethylene oxide with a partial ester derived from fatty acid and a hexitol anhydride (*e.g.,* polyoxyethylene sorbitan monooleate). The aqueous suspension can also contain one or more preservatives such as ethyl or n-propyl p-hydroxybenzoate, one or more coloring agents, one or more flavoring agents and one or more sweetening agents, such as sucrose, aspartame or saccharin. Formulations can be adjusted for osmolarity.

Oil suspensions can be formulated by suspending relaxin in a vegetable oil, such as arachis oil, olive oil, sesame oil or coconut oil, or in a mineral oil such as liquid paraffin. The oil suspensions can contain a thickening agent, such as beeswax, hard paraffin or cetyl alcohol. Sweetening agents can be added to provide a palatable oral preparation. These formulations can be preserved by the addition of an antioxidant such as ascorbic acid.

Dispersible powders and granules of the disclosure suitable for preparation of an aqueous suspension by the addition of water can be formulated from relaxin in admixture with a dispersing, suspending and/or wetting agent, and one or more preservatives. Suitable dispersing or wetting agents and suspending agents are exemplified by those disclosed above. Additional excipients, for example sweetening, flavoring and coloring agents, can also be present.

The pharmaceutical formulations of the disclosure can also be in the form of oil-in-water emulsions. The oily phase can be a vegetable oil, such as olive oil or arachis oil, a mineral oil, such as liquid paraffin, or a mixture of these. Suitable emulsifying agents include naturally-occurring gums, such as gum acacia and gum tragacanth, naturally occurring phosphatides, such as soybean lecithin, esters or partial esters derived from fatty acids and hexitol anhydrides, such as sorbitan mono-oleate, and condensation products of these partial esters with ethylene oxide, such as polyoxyethylene sorbitan mono-oleate. The emulsion can also contain sweetening and flavoring agents. Syrups and elixirs can be formulated with sweetening agents, such as glycerol, sorbitol or sucrose. Such formulations can also contain a demulcent, a preservative, a flavoring or a coloring agent.

### Administration and Dosing Regimens

The formulations containing pharmaceutically active relaxin or pharmaceutically effective relaxin agonist used in the methods of the disclosure can be administered in any conventionally acceptable way including, but not limited to, intravenously, subcutaneously, intramuscularly, sublingually, topically, orally, via inhalation, and by wearable infusion pump. Administration will vary with the pharmacokinetics and other properties of the drugs and the patients' condition of health. General guidelines are presented below.

The methods of the disclosure produce hemodynamic effects consistent with vasoldialtion, including improved parameters reflecting renal function in subjects with stable compensated chronic HF. The amount of relaxin alone or in combination with another agent or drug or agents and drugs that is adequate to accomplish this is considered the therapeutically effective dose. The dosage schedule and amounts effective for this use, *i.e.,* the "dosing regimen," will depend upon a variety of factors, including the stage of the disease or condition, the severity of the disease or condition, the severity of the adverse side effects, the general state of the patient's health, the patient's physical status, age and the like. In calculating the dosage regimen for a patient, the mode of administration is also taken into consideration. The dosage regimen must also take into consideration the pharmacokinetics, *i.e.,* the rate of absorption, bioavailability, metabolism, clearance, and the like. Based on those principles, relaxin can be used to treat human subjects diagnosed with symptoms of heart failure to maintain stable compensated chronic HF.

The disclosure provides relaxin and additional drugs including antiplatelet therapy, beta-blockers, diuretics, nitrates, hydralazine, inotropes, digitalis, and angiotensin-converting enzyme inhibitors or angiotensin receptor blockers for simultaneous, combined, separate or sequential administration. The disclosure also provides the use of antiplatelet therapy, beta-blockers, diuretics, nitrates, hydralazine, inotropes, digitalis, and angiotensin-converting enzyme inhibitors or angiotensin receptor blockers in the manufacture of a medicament for managing stable compensated chronic HF, wherein the medicament is prepared for administration with relaxin.

Further contemplates is the use of relaxin in the manufacture of a medicament for managing stable compensated chronic HF, wherein the patient has previously (*e.g*., a few hours before, one or more days, weeks, or months, or years before, *etc.*) been treated with antiplatelet therapy, beta-blockers, diuretics, nitrates, hydralazine, inotropes, digitalis, and angiotensin-converting enzyme inhibitors or angiotensin receptor blockers. In one embodiment, one or more of the drugs such as, antiplatelet therapy, beta-blockers, diuretics, nitrates, hydralazine, inotropes, digitalis, and angiotensin-converting enzyme inhibitors or angiotensin receptor blockers are still active *in vivo* in the patient. The disclosure also provides the use of antiplatelet therapy, beta-blockers, diuretics, nitrates, hydralazine, inotropes, digitalis, and angiotensin-converting enzyme inhibitors or angiotensin receptor blockers in the manufacture of a medicament for managing stable compensated chronic HF, wherein the patient has previously been treated with relaxin.

The state of the art allows the clinician to determine the dosage regimen of relaxin for each individual patient. As an illustrative example, the guidelines provided below for relaxin can be used as guidance to determine the dosage regimen, *i.e.,* dose schedule and dosage levels, of formulations containing pharmaceutically active relaxin administered when practicing the methods of the disclosure. As a general guideline, it is expected that the daily dose of pharmaceutically active H2 human relaxin (*e.g*., synthetic, recombinant, analog, agonist, *etc*.) is typically in an amount in a range of about 10 to 960 mcg/kg of subject body weight per day. In one embodiment, the dosages of relaxin are 10, 30, and 100 mcg/kg/day. In another embodiment, these dosages result in serum concentrations of relaxin of about 3, 10, and 30 ng/ml, respectively. In another embodiment, the dosages of relaxin are 240, 480, and 960 mcg/kg/day. In another embodiment, these dosages result in serum concentrations of relaxin of about 75, 150, and 300 ng/ml, respectively. In another embodiment, the administration of relaxin is continued as to maintain a serum concentration of relaxin from about 0.5 to about 500 ng/ml, more preferably from about 3 to about 300 ng/ml. Thus, the methods of the present disclosure include administrations that result in these serum concentrations of relaxin. These relaxin concentrations can ameliorate or reduce decompensation events such as dyspnea, hypertension, high blood pressure, arrhythmia, reduced renal blood flow, renal insufficiency and mortality. Furthermore, these relaxin concentrations can ameliorate or reduce neurohormonal imbalance, fluid overload, cardiac arrhythmia, cardiac ischemia, risk of mortality, cardiac stress, vascular resistance, and the like. Depending on the subject, the relaxin administration is maintained for a specific period of time or for as long as needed to maintain stability in the subject.

The duration of relaxin treatment can be indefinitely for some subjects, or preferably kept at a range of about 4 hours to about 96 hours depending on the patient, and one or more optional repeat treatments as needed. For example, with respect to frequency of administration, relaxin administration can be a continuous infusion lasting from about 8 hours to 48 hours of treatment. Relaxin can be given continuously or intermittent via intravenous or subcutaneous administration (or intradermal, sublingual, inhalation, or by wearable infusion pump). For intravenous administration, relaxin can be delivered by syringe pump or through an IV bag. The IV bag can be a standard saline, half normal saline, 5% dextrose in water, lactated Ringer's or similar solution in a 100, 250, 500 or 1000ml IV bag. For subcutaneous infusion, relaxin can be administered by a subcutaneous infusion set connected to a wearable infusion pump. Depending on the subject, the relaxin administration is maintained for as specific period of time (e.g. 4, 8, 12, 24, and 48 hours) or for as long as needed (e.g. daily, monthly, or for 7, 14, 21 days etc.) to maintain stability in the subject.

Some subjects are treated indefinitely while others are treated for specific periods of time. It is also possible to treat a subject on and off with relaxin as needed. Thus, administration can be continued over a period of time sufficient to maintain a stable compensated chronic HF resulting in an amelioration or reduction in acute cardiac decompensation events, including but not limited to, dyspnea, hypertension, high blood pressure, arrhythmia, reduced renal blood flow and renal insufficiency. The formulations should provide a sufficient quantity of relaxin to effectively ameliorate and stabilize the condition. A typical pharmaceutical formulation for intravenous administration of relaxin would depend on the specific therapy. For example, relaxin may be administered to a patient through monotherapy (*i.e.,* with no other concomitant medications) or in combination therapy with another medication such as antiplatelet therapy, beta-blockers, diuretics, nitrates, hydralazine, inotropes, digitalis, and angiotensin-converting enzyme inhibitors or angiotensin receptor blockers or other drug. In one embodiment, relaxin is administered to a patient daily as monotherapy. In another embodiment, relaxin is administered to a patient daily as combination therapy with another drug. Notably, the dosages and frequencies of relaxin administered to a patient may vary depending on age, degree of illness, drug tolerance, and concomitant medications and conditions. In a further embodiment relaxin is administered to a patient with the ultimate goal to replace, reduce, or omit the other medications to reduce their side effects and to increase or maintain the therapeutical benefit of medical intervention using relaxin in order to optimally maintain a stable, compensated, and chronic heart failure.

### EXPERIMENTAL

The following specific examples are intended to illustrate the disclosure and should not be construed as limiting the scope of the claims.

Abbreviations: AUC (area under the curve); BNP (brain natriuretic peptide); (BP) blood pressure; BUN (blood urea nitrogen); CHF (congestive heart failure); CI (cardiac index); CO (cardiac output); CrCl (creatine clearance); DBP (diastolic blood pressure); dL (deciliters); eGFR (estimated glomerular filtration rate); HF (heart failure); hr (hour); HR (heart rate); ICU (intensive care unit); IV (intravenous); kg (kilogram); L (liter); LVEDP (left ventricular end diastolic pressure); LVEF (left ventricular ejection fraction); mcg or µg(microgram); mEq (milliequivalents); MI (myocardial infarction); mIU (milli-international units); mL (milliliter); NYHA (New York Heart Association); PAH (para-aminohippurate); PAP (pulmonary arterial pressure); PCWP (pulmonary capillary wedge pressure); PD (pharmacodynamic); RAP (right atrial pressure); RBBB (right bundle branch block); RBF (renal blood flow); rhRlx or rhRLX (recombinant human relaxin); Rlx or RLX (relaxin); RR (respiratory rate); SBP (systolic blood pressure); SI (stroke index); sMDRD (simplified Modification of Diet in Renal Disease); SQ (subcutaneous SQ); SVR (systemic vascular resistance); T (temperature); VAS (visual analog scale); VF (ventricular fibrillation); VT (ventricular tachycardia); and WHF (worsening heart failure).

### EXAMPLE 1

### Administration of Relaxin to Chronic Heart Failure Patients

*Overview.* In this open-label study 16 patients with chronic and stable compensated congestive HF were enrolled in three dose-ascending cohorts of recombinant intravenous relaxin (10 to 960mcg/kg/day) over 24 hours. Relaxin produced increases in cardiac index and stroke volume, and a decrease in pulmonary wedge pressure and NT-pro BNP. It improved markers of renal function, with a small rebound at the highest dose during post-infusion. This study indicates that relaxin has beneficial hemodynamic, neurohumoral, and renal effects in stable compensated HF patients without relevant adverse effects, and it makes relaxin a major candidate drug to keep stable compensated chronic heart failure in patients under control. A maximally tolerated dose was determined.

*Design of Study.* In this single-center, open-label study, 16 patients who fulfilled inclusion and but not exclusion criteria for chronic and stable compensated congestive HF were sequentially allocated to three ascending cohorts of intravenous (IV) relaxin. Main *inclusion criteria* included: Age >18 years; NYHA CHF Class II-III without restriction on etiology; left ventricular ejection fraction < 35% within 6 months of enrollment; receiving established oral HF therapy expected to remain unchanged during the study period. Main *exclusion criteria* included: Wedge pressure < 16 mmH or CI > 2.5 L/min/m2; acute coronary syndrome (within 4 weeks) or recent myocardial infarction or cardiac surgery (within 6 months); AHF requiring intravenous therapy at baseline; systolic blood pressure < 85 mmHg; uncorrected valvular heart disease, except for relative mitral and/or tricuspid valve insufficiency; obstructive or restrictive cardiomyopathy; recent episode of ventricular tachycardia or fibrillation (within 4 weeks); recent stroke (within 3 months); creatinine > 2.0 mg/dl or serum transaminases and/or total bilirubin > 2.5 times the upper limit of normal at screening visit; history of endometriosis.

*Design of Drug.* The study drug was relaxin (produced by recombinant technology). Recombinant relaxin is identical to the native human hormone H2 relaxin. The dose escalation was as follows: *Group A* included sequential treatment for 8 hours each with dosages equivalent to 10, 30, and 100 mcg/kg/day. *Group B* included sequential treatment for 8 hours each with dosages equivalent to 240, 480, and 960 mcg/kg/day. *Group* C received 24 hours of treatment with 960 mcg/kg/day. Escalation from Group A to Group B was done after examining safety and tolerability of the doses used in Group A. Escalation from Group B to C occurred after the safety and tolerability of the highest dose in Group B (960 mcg/kg/day) was determined.

*Study Procedures, Endpoints and Statistical Analysis.* Patients were monitored in the intensive care unit for the infusion and post-infusion periods (24 hours either). Hemodynamic measurements, including CI, SVR, PCWP, SBP, RAP, and PVR, were serially performed using Swan-Ganz and arterial catheters. Likewise, clinical and laboratory monitoring was carried out serially throughout infusion and post-infusion and on Day 9 after beginning of infusion. An additional 30-day evaluation for serious adverse events was performed by phone. Endpoints included hemodynamic and neurohumoral (NT-pro BNP levels) changes from baseline, as well as monitoring of vital signs, electrocardiogram, serum chemistry, and hematology parameters throughout the study. For statistical analysis, an error probability of P<.05 was regarded as significant. Baseline values were compared using the Kruskal-Wallis ANOVA on ranks followed by Dunn's test. Differences over time within the single groups (hemodynamics, renal parameter, and peptide levels) were analyzed with a Friedman Repeated Measures Analysis of Variance on Ranks followed by Dunn's test for comparison against the baseline.

*Demographics and Safety.* All patients were on standard HF medication(s) and showed markedly depressed left ventricular systolic function due to coronary artery disease, hypertension, dilated cardiomyopathy, or (in one case) corrected valvular heart disease. All subjects completed dosing, and all doses of relaxin were well tolerated. See Table 1 below. There were no infusion-related clinically significant adverse events; the highest dose tested in Groups A and B, 960mcg/kg/d, was therefore chosen to be administered in Group C over 24 hours. Three weeks after study drug infusion, one adverse event occurred (moderate angina pectoris without any sign of progressive coronary artery disease upon angiographic evaluation), which was deemed not related to relaxin. There were seven adverse events not related to study drug reported during dosing through Day 9, *i.e.,* two patients complained of mild angina pectoris (not including the SAE), and one each complained of weakness, insomnia, headache, benign prostatic hypertrophy, and mild hemoptysis. The hemoptysis event was clearly induced by advancing the Swan-Ganz catheter into wedge position and recovered spontaneously.

**TABLE 1 Study Subjects**

| **GROUP** | **SUBJECT** | **MEDICATION** | **LVEF** |
|---|---|---|---|
| A | Male, Caucasian, 79 years, CAD | ASA, STA, BB, ACEI, AA, DIG, D | 31% |
| A | Male, Caucasian, 65 years, CAD | ASA, CLO, STA, BB, ACEI, NIT, D | 33% |
| A | Male, Caucasian, 82 years, CAD | ASA, CLO, STA, BB, ACEI, D | 23% |
| A | Female, Caucasian, 73 years, Hypertension | ASA, BB, ACEI, D | 28% |
| B | Male, Caucasian, 68 years, Hypertension | BB, SAR, AA, DIG, D | 28% |
| B | Male, Caucasian, 60 years, DCMP | BB, ACEI, AA, D | 30% |
| B | Male, Caucasian, 73 years, CAD | ASA, CLO, STA, BB, ACEI, AA | 23% |
| B | Male, Caucasian, 47 years, Hypertension | STA,BB, ACEI, AA, DIG, D | 28% |
| B | Male, Caucasian, 50 years, CAD | STA, CLO, BB, ACEI, AA, NIT, CCB, D | 22% |
| B | Male, Caucasian, 63 years, Hypertension | BB, ACEI, AA, NIT, D, AMD | 26% |
| C | Male, Caucasian, 69 years, DCMP | STA, BB, SAR, AA, DIG, D | 25% |
| C | Male, Caucasian, 78 years, CAD | ASA, CLO, STA, BB, ACEI, CCB, D | 29% |
| C | Male, Caucasian, 64 years, CAD | ASA, STA, BB, ACEI, SAR, D | 23% |
| C | Male, Caucasian, 72 years, Valvular CHF* | BB, ACEI, DIG, D, AMD | 26% |
| C | Male, Caucasian, 64 years, CAD | ASA, BB, ACEI, AA, NIT, D | 20% |
| C | Male, Caucasian, 64 years, CAD | ASA, CLO, STA, BB, SAR, AA, CCB, D | 24% |

| | | | |
|---|---|---|---|
| LVEF = left ventricular ejection fraction; ASA = acetylsalicylic acid; CLO = clopidogrel; STA = statin; BB = beta blocker; ACEI = angiotensin-converting enzyme inhibitor; SAR = sartan; CCB = calcium channel blocker; AA = aldosterone antagonist; DIG = digitalis; NIT = nitrate; D = diuretic; AMD = amiodarone. *after surgical correction | | | |

*Baseline Hemodynamics and Kidney Function.* The patients in group A had a trend toward more abnormal baseline parameters, while group B appeared the least abnormal. Patients in Group B had a significantly lower SVR than Group A patients, and Group C demonstrated significantly higher PVR values than Group B. Concerning renal parameters, patients in Group C tended to have higher creatinine and BUN values than Group A and B patients, although this did not reach significance. Likewise, the trend towards lower NT-pro BNP values seen in Group B was not significant. See Table 2 below.

**TABLE 2 Baseline Values of Study Groups**

| | **Group A** | **Group B** | **Group C** |
|---|---|---|---|
| **CI** (1/min/m²) | 2.1 ± 0.2 | 2.3 ± 0.1 | 2.1 ± 0.1 |
| **PCWP** (mm Hg) | 20 ± 1 | 19 ± 2 | 20 ± 2 |
| **SVR** (dyn s⁻¹ cm⁵) | 1518 ± 134 | 1010 ± 52* | 1254 ± 98 |
| **PVR** (dyn s⁻¹ cm⁵) | 194 ± 34 | 128 ± 12 | PVR (dyn s-1 cm5) |
| **SBP** (mm Hg) | 128 ± 9 | 109 ± 5 | 118 ± 8 |
| **MPAP** (mm Hg) | 30 ± 1 | 27 ± 2 | 33 ± 4 |
| **RAP** (mm Hg) | 8 ± 2 | 12 ± 2 | 10 ± 2 |
| **HR** (bpm) | 71 ± 6 | 68 ± 2 | 66 ± 5 |
| **Creatinine** (mg/dl) | 1.08 ± 0.13 | 1.19 ± 0.10 | 1.46 ± 0.16 |
| **BUN** (mg/dl) | 39 ± 7 | 47 ± 8 | 72 ± 12 |
| **NT-pro BNP** (pg/ml) | 3715 ± 1046 | 2273 ± 648 | 2693 ± 501 |

| | | | |
|---|---|---|---|
| CI = cardiac index; PCWP = pulmonary capillary wedge pressure; SVR/PVR = systemic/pulmonary vascular resistance; SBP = systolic blood pressure; MPAP = mean pulmonary artery pressure; RAP = right atrial pressure; HR = heart rate; BUN = blood urea nitrogen. P < 0.05, *A vs. B, # B vs. C | | | |

*Infusion and Post-Infusion.* The values for CI (Figure 4) tended to increase with rising relaxin doses in Group A. In Group B, CI tended to rise during the first 8-hour period (240 mcg/kg/d), then declined at 480 mcg/kg/d, and finally, it increased significantly at 960 mcg/kg/d. In Group C, the latter dose produced a significant and sustained elevation of CI, with absolute increases of up to 0.81/min/m2. This remarkable effect gradually wore off within the first 8 hours post-infusion. It is noteworthy that this CI increase was exclusively attributable to a heightened stroke volume since heart rate did not change in any group (Figure 5). The changes of CI were paralleled by corresponding reciprocal changes of SVR (Figure 6), which reached statistical significance in Group C. Concerning PCWP (Figure 7), values dropped significantly at 30 and 100/kg/d in Group A. In Group B, PCWP appeared to fall during the first 8 hours but then returned to baseline level despite increasing Relaxin doses. Again, 960 mcg/kg/d of relaxin given over 24 hours in Group C induced a clear and significant effect, with absolute decreases in PCWP of approximately 4 mm Hg. In general, significant alterations of SBP (Figure 8) were not observed. In Group A, which had demonstrated a trend to higher SBP at baseline (128 ± 9 mm Hg, cp. Table 2), SBP apparently tended to decline whereas in Groups B and C, there was no relevant change. The time course of NT-pro BNP (Figure 9) corresponded well with the hemodynamic response seen in the different groups: we observed significant declines in Groups A and C but no change in Group B, with a trend towards even higher values in this group on Day 9. Finally, creatinine values decreased during Relaxin infusion in all groups (Figure 10), the effect becoming significant in groups A and C. With increasing dosages in Groups B and C, the values determined on Day 9 seemed to indicate a certain rebound effect although none of the patients developed renal adverse events or required medical intervention. The time course of BUN values paralleled that determined for creatinine. Throughout the study, relaxin did not evoke any abnormalities regarding vital signs, ECG, serum chemistry, and hematology parameters.

*Findings.* This pilot study is the first to explore the use of relaxin in chronic congestive HF patients. The main aim of the pilot study was to explore the safety and tolerability of the relaxin formulation, as well as its dose-response in stable chronic HF patients. The study demonstrated the following. 1) Over a wide dose range (10 - 960 mcg/kg/day), relaxin showed no relevant adverse effects and was well-tolerated. 2) Relaxin produced beneficial hemodynamic effects (e.g., a decrease in vascular resistance, an increase in cardiac index attributable to elevated stroke volume, and a decrease in wedge pressure, without inducing hypotension). 3) Relaxin administration was associated with early decreases in creatinine and BUN. 4) In patients receiving the highest relaxin dose (960 mcg/kg/day), a potentially dose-limiting increase in post-treatment creatinine level was observed. This suggests that a dose of 240 - 480 mcg/kg/day is likely the maximally tolerated dose (MTD) of IV relaxin in this population, in the absence of physician supervision. In general, Groups A and C demonstrated comparable responses to infusions of relaxin while Group B did not. This may be accounted for by baseline differences: patients in Group B were already maximally vasodilated as evidenced by their significantly lower SVR and the trends towards lower SBP and NT-pro BNP values (Table 2). In those patients, potential further vasodilation by relaxin ("over-vasodilatation"), likely precipitated relevant counter-regulatory responses preventing assessment of the full potential hemodynamic effects of the drug, especially in the medium dose (480 mcg/kg/day).

The patients in this study were stable, but showed signs of advanced heart failure. They would most likely fall into that quartile of the ADHERE registry, which has SBP values lower than 120 mmHg and, correspondingly, the worst outcome. Judging from the fact that hypotension on hospital admission as well as therapy-related hypotension are known or suspected to worsen prognosis of AHF, it is crucial to assess the response of SBP to relaxin therapy. In this study, no symptomatic hypotension was observed despite significant reductions in systemic vascular resistance. There appears to be a trend towards SBP decrease in Group A but this can be ascribed to 1 out of 4 individuals who experienced a sustained yet asymptomatic fall of SBP (from about 120/65 mm Hg to about 100/50 mm Hg). No significant SBP changes in Groups B (less severe group) and C despite rising relaxin doses were recorded. Nevertheless, the lack of significant hypotension is important to note. Moreover, in patients presenting with higher blood pressures than those recorded in this study, the hypotensive effect of relaxin is likely more pronounced. These findings are corroborated by others (see Debrah et al. (2005) Hypertension 46:745-50), *i.e.,* in both hypertensive and normotensive rats, rhRlx elicited an increase in CI and a decrease in SVR without affecting mean arterial pressure. If rhRlx was a non-specific vasodilator one would expect a fall of SBP partly offset by elevated stroke volume.

None of the treatment groups showed a significant decrease in right atrial pressure (Figure 11) despite of the remarkable and significant drop of PVR in Group C (∼30 %) and the significant fall of PCWP seen in Groups A and C (Figure 7). This indicates that relaxin might promote venous return to maintain central venous filling pressure. Some investigators (Edouard et al. (1998) Am J. Physiol. 274:H1605-H1612) reported an elevation of venous tone in the lower limb beginning in the first trimester of pregnancy. Relaxin, first discovered as a pregnancy hormone, is the key mediator for the renal adaptation to pregnancy (see Novak et al. (2001) J. Clin. Invest. 107:1469-75). Moreover, the hemodynamic pattern observed here, increased CI and decreased SVR but no relevant fall of SBP, resembles that seen in pregnancy (see Slangen et al. (1996) Am J. Physiol. 270:H1779-H1784).

Infusion of relaxin resulted in an improvement in parameters reflecting renal function (creatinine, BUN) in most patients, an effect that became significant in Groups A and C. Because relaxin has been shown to increase glomerular filtration (GFR) and renal blood flow in animals (see Novak, *supra,* and Jeyabalan et al. (2003) Circ. Res. 93:1249-57) a similar mechanisms may be active here. Even so, an understanding of the mechanism is not necessary to make and use embodiments of the present disclosure. Although relaxin-induced increases in GFR have not been measured in humans, a small open-label study has shown that relaxin increases renal blood flow in healthy volunteers (Smith et al. (2006) J. Am. Soc. Nephrol. 17:3192-7). In clinical trials in scleroderma patients (Seibold et al. (2000) Ann. Intern. Med. 132:871-9), relaxin led to a sustained amelioration of predicted creatinine clearance. Concerning the post-infusion period, an increase on Day 9 in creatinine and BUN was recorded following the administration of IV relaxin in doses equivalent to 960 mcg/kg/day, which spontaneously resolved by Day 30. None of the creatinine increases were beyond ε 0.5 mg/dl, which is the most widely used definition for worsening renal function, and there were no clinical renal adverse events. In fact, 960mcg/kg/day dose was associated with remarkable increases in CI. It is possible that this high dose stimulated counter-regulatory systems leading to rebound hemodynamic responses associated with reduced kidney perfusion and a late increase in BUN and creatinine. Indeed, the 32- and 48-hour hemodynamic measurements following the 960 mcg/kg/day dose, both the wedge and right atrial pressures tended to be higher than at baseline, an effect not seen with other doses. These findings suggest that IV relaxin at a dose of 960 mcg/kg/day may have some dose limiting adverse effects both on hemodynamics and renal function. The maximally tolerated dose (MTD) without physician supervision could hence be determined by the present study to be in the range of 240 - 480 mcg/kg/day. Interestingly, doses of relaxin in the range of 10 to 100 mcg/kg/day appeared to have a more pronounced effect than higher doses on PCWP, SBP, and NT-pro-BNP, whereas higher doses in the range of 240 to 960 mcg /kg/day tended to have a greater effect on CO and CI. Doses in the lower range may produce mostly venous vasodilation (lower PCWP without relevant change in CO/CI), whereas higher doses may produce more arterial vasodilation (higher CO/CI).

**TABLE 3 Baseline and Change from Baseline in Cardiac and Hemodynamic Parameters**

| | **Group A** | | | **Group B** | | | **Group C** | |
|---|---|---|---|---|---|---|---|---|
| Infusion rate (mcg / kg / d) | 10 | 30 | 100 | 240 | 480 | 960 | 960 | 960 |
| Infusion duration (hr) | 8 | 8 | 8 | 8 | 8 | 8 | 8 | 24 |
| HR Baseline (beats/min) | 71.5 ± 11.2 | | | 67.5 ± 6.1 | | | 66.2 ± 11.5 | |
| Change from baseline | 3.3 ± 7.6 | 2.3 ± 16.2 | 0.5 ± 1.3 | 1.7 ± 6.7 | -1.8 ± 4.5 | 1.8 ± 3.4 | 2.5 ± 4.3 | 0.67 ± 3.6 |
| SBP Baseline (mm Hg) | 128.5 ± 18.0 | | | 109.2 ± 11.6 | | | 118.3 ± 20.0 | |
| Change from baseline | -12.5± 9.3 | -5.3 ± 16.8 | -13.0 ± 8.3 | -2.5± 7.4 | 2.2 ± 5.6 | -3.0 ± 5.1 | -1.3 ± 4.7 | -7.3 ± 9.5 |
| DBP Baseline (mm Hg) | 59.5 ± 5.2 | | | 55.3 ± 6.1 | | | 55.3 ± 9.7 | |
| Change from baseline | -3.0 ± 4.2 | 2.5 ± 10.9 | -5.0 ± 3.7 | 3.0 ± 6.0 | 0.0 ± 7.2 | -0.3 ± 6.8 | -4.8 ± 5.4 | -6.5 ± 7.5 |
| CI Baseline (L / min / m2) | 2.1 ± 0.4 | | | 2.3 ± 0.2 | | | 2.1 ± 0.2 | |
| Change from baseline | 0.08 ± 0.3 | 0.25 ± 0.4 | 0.25 ± 0.2 | 0.28 ± 0.2 | 0.17 ± 0.3 | 0.25 ± 0.3 | 0.43 ± 0.3 | 0.67 ± 0.15* |
| CO Baseline (L / min) | 4.0 ± 0.7 | | | 4.8 ± 0.5 | | | 4.1 ± 0.6 | |
| Change from baseline | 0.13 ± 0.5 | 0.45 ± 0.6 | 0.45 ± 0.2 | 0.62 ± 0.4 | 0.33 ± 0.7 | 0.48 ± 0.7 | 0.85 ± 0.5 | 1.25 ± 0.03* |
| SVR Baseline (dynes*sec/cm5) | 1518 ± 268 | | | 1011 ± 127# | | | 1254 ± 239 | |
| Change from baseline | -143 ± 140 | -171 ± 159 | -288 ± 92 | -150 ± 138 | -12 ± 219 | -102 ± 248 | -255 ± 188 | -395 ± 217* |
| RAP Baseline (mm Hg) | 12.8 ± 10.0 | | | 11.7 ± 5.0 | | | 10.3 ± 6.1 | |
| Change from baseline | 0.8± 0.9 | -1.0 ± 1.8 | -0.8 ± 1.2 | 0.3 ± 1.4 | 0.5 ± 1.1 | 0.8 ± 1.2 | -1.2 ± 2.9 | -1.5 ± 2.7 |
| PAP Baseline (mm Hg) | 29.8 ± 2.8 | | | 27.5 ± 5.4 | | | 32.7 ± 8.9 | |
| Change from baseline | -3.75 ± 4.1 | -4.0 ± 5.5 | -4.5 ± 4.2 | -1.7 ± 1.6 | 1.7 ± 2.7 | 0.3 ± 3.7 | -3.2 ± 3.1 | -5.0 ± 3.4 |
| PCWP Baseline (mm Hg) | 19.8 ± 1.7 | | | 19.3 ± 4.8 | | | 19.8 ± 4.2 | |
| Change from baseline | -1.8 ± 1.9 | -5.0 ± 2.6* | -3.5 ± 3.7 | -1.5 ± 1.9 | 0.33 ± 2.6 | -0.17 ± 2.7 | -2.5 ± 3.5 | -3.5 ± 3.0 |
| PVR Baseline (dynes*sec/cm5) | 193.5 ± 67.2 | | | 127.7 ± 29.0 | | | 267.2 ± 168.1 § | |
| Change from baseline | -17.3 ± 60.5 | -0.5 ± 56.7 | -25.3 ± 28.9 | -14.8 ± 16.9 | 5.5 ± 35.2 | 3.3 ± 27.5 | -58.5 ± 69.4 | -82.5 ± 55.5* |

| | | | | | | | | |
|---|---|---|---|---|---|---|---|---|
| CI, cardiac index; CO, cardiac output; PCWP, pulmonary capillary wedge pressure; SVR/PVR, systemic/pulmonary vascular resistance; SBP, systolic blood pressure; DBP, diastolic blood pressure; PAP, mean pulmonary artery pressure; RAP, right atrial pressure; HR, heart rate. Values are mean ± SD. *P < .05 compared with baseline. #Group A vs. B. §Group B vs. C. | | | | | | | | |

*Conclusion.* The aim of the pilot study was to determine safety and tolerability of relaxin when administered to chronic heart failure patients and the pharmacodynamic dose response to intravenous relaxin in patients with established chronic HF. This study was the first therapeutic use of relaxin in human heart failure. Multiple conclusions were drawn from the chronic HF clinical trial. Over the entire dose range, relaxin showed no relevant adverse effects. Relaxin produced beneficial hemodynamic effects paralleled by NT-pro BNP changes, *i.e.,* an increase in cardiac index that is attributed to elevated stroke volume and a decrease in pulmonary wedge pressure and systemic vascular resistance, without inducing hypotension. Relaxin rapidly improved markers of renal function (creatinine, BUN). At the highest dose, it evoked a small and spontaneously recovering increase in renal markers during post-infusion. Hence, the maximally tolerated dose of relaxin without physician supervision in the present study is 240-480 mcg/kg/day.

In summary, IV relaxin administered to patients with chronic stable heart failure led to vasodilation followed by a decrease in wedge pressure and an increase in stroke volume. Significant decreases in blood pressure were not observed due to the size of the cohort, characteristics of the patients enrolled and/or additional properties of relaxin. In addition to the hemodynamic effects, IV relaxin induced an early decrease in creatinine and BUN that could be a positive attribute when administered to patients with AHF. An MTD of relaxin was identified in the present study based on late increases in wedge and right atrial pressures, as well as creatinine and BUN after study drug discontinuation at the highest dose of IV relaxin (960 mcg/kg/day). Thus, during this first therapeutic use of intravenous relaxin in human HF patients, the safety and efficacy profile of relaxin indicates that it is effective in the treatment of chronic and stable compensated HF.

### EXAMPLE 2

### Administration of Relaxin to Systemic Sclerosis Patients

*Overview.* Clinical trials with relaxin have also been conducted on systemic sclerosis patients. 257 human subjects who suffer from systemic sclerosis, a serious fibrotic disease, have been treated with relaxin by continuous and subcutaneous (SQ) infusion for six months. The results, which include extensive and long term safety information, have shown that these patients did not experience any serious hypotensive events as a result of relaxin (Figure 12), confirming the later CHF findings. The systemic sclerosis trials showed that relaxin administration was associated with stable decreases in blood pressure, with no serious episodes of hypotension, and a statistically significant increase in predicted creatinine clearance (see Figure 13). These findings support the hypothesis that relaxin administration was associated with balanced systemic and renal vasodilation.

In addition, 570 human subjects have been treated with relaxin in 19 completed trials. These subjects included patients with fibromyalgia, women undergoing egg donation, pregnant women at term, healthy female and male volunteers, healthy adults undergoing orthodontic therapy, and systemic sclerosis patients.

*Findings and Conclusion.* Relaxin can be administered safely in subjects with a variety of underlying conditions. In a number of these trials, data suggested that relaxin causes balanced systemic and renal vasodilation.

Various modifications and variations of the present disclosure will be apparent to those skilled in the art without departing from the scope and spirit of the disclosure. Although the disclosure has been described in connection with specific preferred embodiments, it should be understood that the claims should not be unduly limited to such specific embodiments. Indeed, various modifications of the described modes for carrying out the disclosure, which are understood by those skilled in the art are intended to be within the scope of the claims.

## Claims

1. A pharmaceutically active H2 human relaxin for use in treating chronic heart failure comprising administering said H2 human relaxin to a human subject with Class II or Class III heart failure according to New York Heart Association classification at the onset of administration, in an amount therapeutically effective to reduce the frequency or duration of hospitalization of the subject compared to treatment without said relaxin,
wherein the frequency of decompensation is reduced compared to treatment without relaxin, and, wherein said relaxin is administered at a fixed dose of 10 to 960 µg/kg/day.

2. The pharmaceutically active relaxin of claim 1, wherein H2 relaxin is administered at 10, 30, 100, 240, 480 or 960 µg/kg/day.

3. The pharmaceutically active relaxin according to any of the preceding claims, wherein said relaxin is administered by infusion for at least 24 hours.

4. The pharmaceutically active relaxin of any of the preceding claims, wherein administration further results in decreasing one or more of systemic vascular resistance, pulmonary capillary wedge pressure, pulmonary vascular resistance, blood urea nitrogen, creatinine and circulating N-terminal prohormone brain natriuretic peptide.

5. The pharmaceutically active relaxin of any of the preceding claims, wherein said subject is receiving one or more of a beta-blocker, a diuretic and an anti-angiotensin therapy (angiotensin-converting enzyme inhibitor or angiotensin receptor blocker) at the onset of the administration.

6. The pharmaceutically active relaxin of any of the preceding claims, wherein said subject does not have acute heart failure requiring hospitalization at the onset of the administration.

7. The pharmaceutically active relaxin of any of the preceding claims, wherein the subject has a systolic blood pressure about 85 mm Hg or greater at the onset of the administration.
